# EUROPEAN PATENT APPLICATION

(11) **EP 4 570 262 A1**
(43) Date of publication of application: **18.06.2025**
(21) Application number: 23217219.7
(22) Date of filing: 15.12.2023
(51) Int. Cl.: A61K 39/00, A61P 35/00

(54) **CHIMERIC ANTIGEN RECEPTOR DIRECTED AGAINST THE CHEMOKINE RECEPTOR CCR7**

(71) Applicant: Max-Delbrück-Centrum für Molekulare Medizin in der Helmholtz-Gemeinschaft, 13125 Berlin (DE)
(72) Inventor: Höpken, Uta E., 14057 Berlin (DE); Rehm, Armin, 14057 Berlin (DE); Massaro, Marialucia, 10409 Berlin (DE); Bunse, Mario, 10961 Berlin (DE)
(74) Representative: Hertin und Partner Rechts- und Patentanwälte PartG mbB

(57) **Abstract**

The invention relates to a chimeric antigen receptor polypeptide (CAR) that binds C-C chemokine receptor type 7 (CCR7). The CAR comprises an extracellular antigen-binding domain, comprising an antibody or antibody fragment that binds C-C chemokine receptor type 7 (CCR7) protein, a transmembrane domain, and an intracellular domain. The invention further relates to an isolated nucleic acid molecule encoding a chimeric antigen receptor (CAR) polypeptide of the invention, and a genetically modified cell comprising a nucleic acid molecule and/or expressing a CAR according to the present invention.

## Description

The invention is in the field of recombinant technology and molecular biology, chimeric antigen receptors and cell therapy of medical conditions.

The invention relates to a chimeric antigen receptor polypeptide (CAR) against a C-C chemokine receptor type 7 (CCR7) protein. The invention therefore relates to a chimeric antigen receptor polypeptide (CAR), wherein the CAR comprises an extracellular antigen-binding domain, comprising an extracellular antigen-binding domain, comprising an antibody or antibody fragment that binds C-C chemokine receptor type 7 (CCR7) protein, a transmembrane domain, and an intracellular domain.

The invention further relates to an isolated nucleic acid molecule encoding the CAR of the present invention, or an antigen-binding domain sequence of the present invention. The invention further relates to a genetically modified immune cell comprising a nucleic acid molecule and/or expressing a CAR according to the present invention, and to the treatment of medical conditions employing the CAR, nucleic acids and cells of the present invention.

### BACKGROUND OF THE INVENTION

The C-C chemokine ligand 19 - C-C chemokine receptor 7 (CCL19-CCR7) axis regulates immune responses to viral infections both *in vitro* and *in vivo.* CCR7 is a homeostatic chemokine receptor and is expressed on various immune cells, including double negative (DN) and single positive (SP) thymocytes, naive, central memory, and regulatory T cells (T_{N}, T_{CM}, T_{REG}) as well as naive B cells, CD56⁺CD16⁻ regulatory natural killer (NK) cells, and semi-mature and fully mature dendritic cells (DC), underscoring its significance in guiding these cells to secondary lymphoid organs (Legler, DF et al., Int J Biochem Cell Biol., 2014).

Upon pathogen encounter, CD56+CD16- regulatory NK cells and semi- and mature dendritic cells (DC) upregulate CCR7. Hence, this chemokine receptor regulates immune cell migration and survival to and within secondary lymphoid organs, particularly lymph nodes. These physiological processes are mediated through the binding of CCR7 to its cognate ligands, the chemokines CCL19 and CCL21 , which are constitutively expressed in lymphoid organs by lymphatic vessels, high endothelial venules (HEVs), and fibroblastic stroma cells of the T cell zone. The same processes play a crucial role in the tissue-tropism of leukemia and lymphoma cell dissemination and localization, and distinct blood cancer entities express CCR7 according to their lymphoid origin and maturation status.

In hematological malignancies, particularly leukemias and lymphomas, evidence suggests that CCR7 orchestrates cell trafficking, critically impacting disease progression and treatment outcomes (Cuesta-Mateos C et al., Front Oncol, 2021). CCR7 is highly expressed in a variety of B cell malignancies, including chronic lymphocytic leukemia (CLL), mantle cell lymphoma (MCL), and classical Hodgkin lymphoma (cHD). Strong CCR7 expression has also been detected in T-cell prolymphocytic leukemia (T-PLL) and implicated in acute T-cell leukemia central nervous system (CNS) infiltration. Malignancies of skin-homing T cells, the cutaneous T-cell lymphomas (CTCLs), represent a very heterogeneous group of non-Hodgkin lymphomas encompassing leukemic variants such as Sezary syndrome (SS) and Mycosis fungoides (MF) that also highly express CCR7.

Chronic Lymphocytic Leukemia (CLL), the most common leukemia in Western countries, showcases the significance of CCR7 in cancer pathophysiology. CLL cells, characterized by clonal proliferation of mature B-cells, consistently exhibit CCR7 overexpression. This overexpression, observed in tissues such as peripheral blood, bone marrow, and lymph nodes, is associated with nodal disease involvement. Genetic factors contributing to CCR7 overexpression in CLL remain underexplored. However, studies suggest the involvement of Notch1 mutations, B-cell receptor signaling pathways, and specific integrins. CCR7 facilitates the migration of CLL cells to lymph nodes, promoting interactions with accessory cells and creating a protective microenvironment.

In Mantle Cell Lymphoma (MCL), CCR7 is overexpressed, contributing to the preferential dissemination pattern in MCL patients. Studies reveal higher surface expression of CCR7 in MCL cells than normal B-cells, suggesting altered protein turnover as a potential cause. CCR7-driven migration is implicated in MCL dissemination, and in pre-clinical models, inhibiting CCR7 reduces lymphoma cell viability within tumor masses, highlighting its role in migration and promoting survival.

In classical Hodgkin Lymphoma (cHL), CCR7 is expressed in tumor cell lines and primary tissues, with moderate to high functional expression. Immunohistochemistry studies show strong CCR7 expression in interfollicular cHL zones, while the nodular lymphocyte-predominant variant (NLPHL) is CCR7-negative. CCR7 upregulation in cHL may result from altered pathways, including constitutive activation of AP-1 and NF-κB. Additionally, WNT signaling in cHL contributes to CCR7-mediated migration and angiogenesis. The CCR7 axis recruits both cHL tumor cells and pro-tumorigenic immune cells, including CCR7+ regulatory T-cells, potentially contributing to immune escape mechanisms in cHL.

T cell prolymphocytic leukemia (T-PLL), the most frequent mature T cell leukemia in Western countries, is characterized by aggressive clinical behavior and poor responses to conventional chemotherapies. The disease involves the proliferation of predominantly CD4+ prolymphocytes in various tissues, including the peripheral blood, bone marrow, spleen, liver, lymph nodes, skin, and effusions. CNS involvement is also not uncommon, suggesting a potential role for chemokine receptors in T-PLL dissemination.

While previous evidence did not indicate CCR7 mRNA overexpression in T-PLL samples, it was recently found that CCR7 surface levels are significantly elevated in a high proportion (86.5%) of T-PLL cases. Functional analysis revealed that CCR7, when activated by its ligands CCL19 and CCL21, triggered downstream signaling pathways involving PI3K and ERK, which are relevant in T-PLL pathogenesis. Moreover, CCR7 activation induced chemotaxis, invasion through biological matrices or endothelial cells, and enhanced T-PLL cell survival.

In pre-clinical *in vivo* studies, CCR7 was found to play critical roles in enabling T-PLL cells to access tumor microenvironments in the CNS and lymphoid organs, especially in lymph nodes. CCR7 promotes rapid niche colonization, survival, and proliferation in these environments. Notably, the infiltration of prominent high endothelial venules (HEV) by neoplastic cells in T-PLL suggested that CCL21 serves as a major route for homing into lymphoid tissues, mediating the dissemination of T-PLL cells to different organs.

Initially explored in solid tumors, CAR-T cell therapies show promise in treating blood cancers. Focus areas include Hodgkin's lymphoma (HL), Non-Hodgkin lymphoma (NHL), B-cell lymphomas (BCL), T cell lymphomas (TCL), acute myeloid leukemia (AML), and multiple myeloma (MM).

Ongoing trials explore anti-CD123 and anti-CD33 CAR-T cells for AML and anti-BCMA CAR-T cells for MM. Despite progress, challenges like immune escape mechanisms and toxicities persist, emphasizing the need for continuous research and innovative strategies to enhance the effectiveness of CAR-T cell therapies in hematologic malignancies (Jogalekar MP et al., Front Immunol., 2022). Ongoing phase III clinical trials targeting various antigens, including CD19, CD22, CD30, and BCMA, aim to delineate further the safety and efficacy of CAR-T cell therapies in these diverse blood cancers.

However, CAR-T therapy comes with significant adverse effects, including graft-versus-host disease (GvHD), cytokine release syndrome (CRS), and immune effector cell-associated neurotoxicity syndrome (ICANS). Despite advancements in minimizing these risks through gene editing and other strategies, CAR-T therapy remains associated with individual-specific adverse reactions, relapses, and high costs.

In contrast, NK cells, a distinct class of lymphocytes, offer a promising alternative for immunotherapy. NK cells, which comprise about 10-20% of peripheral blood lymphocytes, are characterized by their ability to recognize and kill tumor cells through various activating receptors. NK cells are defined as CD3-/CD56+ lymphocytes and consist of two subpopulations: the CD56bright subset, comprising approximately 10% of NK cells, primarily located in lymphoid and non-lymphoid tissues, secretes cytokines (IFN-γ, IL-12, IL-15, and IL-18) for immunomodulation, while the CD56dim subset, representing about 90% of NK cells, expresses high levels of FcγIII receptor (CD16a) and possesses abundant granzymes and perforin-containing lytic granules.

Various immunotherapeutic applications of NK cells, including cytokine therapy, adoptive cell therapy (ACT), genetically engineered NK cell therapy (CAR-NK), monoclonal antibody therapy, and NK cell engagers, demonstrate their versatility in combating cancer. CAR-NK therapy, in particular, has gained attention for its improved safety profile compared to CAR-T therapy. Unlike CAR-T, NK cells exhibit inherent advantages, such as superior safety by minimizing the risk of the common adverse effect associated with CAR-T cell therapy - the cytokine release storm (CRS). This is because NK cells secrete different cytokines, such as IFN-gamma and GM-CSF, compared to the pro-inflammatory cytokines involved in CRS. Secondly, using allogeneic sources of NK cells is feasible without inducing GvHD due to the absence of HLA restriction. Thirdly, CAR-NK cells exhibit enhanced killing efficacy by combining their natural cytolytic capacity with engineered killing capabilities. As part of the innate immune system, NK cells naturally exert cytotoxic functions against tumor and virally infected cells without prior sensitization. Activated NK cells eliminate tumor cells through the release of various cytotoxic agents. Lastly, the manufacturing process of CAR-NK cells is more convenient than that of CAR-T cells, as there is no risk of GVHD, and donors can be either HLA-matched or unmatched individuals from diverse sources of NK cells. Clinical trials have demonstrated the potential of CAR NK therapy in treating various blood and solid tumors, presenting a compelling avenue for future cancer immunotherapy research.

To the inventors' knowledge, no alternative anti-CCR7 CAR-NK constructs have been previously described, and no anti-CCR7 antibody studies relevant to the medical approach of the present invention are currently available.

Creative Biolabs offers vectors of anti-CCR7 CARs that are constructed for engineering T cells to target human CCR7. However, these products are for research use only and cannot be used in the treatment or diagnosis of disease, according to the manufacturer.

Multiple ongoing studies are investigating anti-CCR7 antibodies for treating relapsed/refractory CLL and NHLs. Other treatment approaches include anti-CD5-targeting CAR, CD5/CD7 bispecific CAR, biepitopic CAR T cells, anti-CCR4 CAR constructs, standard therapies, and antibodies like Mogamulizumab targeting CCR4 in conditions such as Mycosis fungoides, Sezary syndrome, and adult T cell leukemia/lymphoma.

Preliminary findings from clinical trials evaluating the efficacy and safety of CAR-NK cell therapy have yielded promising results. In a phase I/II study (NCT03056339) involving a limited cohort of subjects (n = 11), 73% of patients exhibited a favorable response after administering HLA-mismatched anti-CD19 CAR-NK cells. This therapeutic intervention was administered to individuals experiencing relapsed or refractory CD19-positive non-Hodgkin's lymphoma or chronic lymphocytic leukemia. Notably, the observed positive effects were attained without consequential toxicities.

Although a number of potential alternative therapies are in development for diseases of pathogenic B and T cells, a significant need remains for providing effective means for addressing such medical disorders.

### SUMMARY OF THE INVENTION

In light of the prior art, the technical problem underlying the invention was providing an agent suitable for treating diseases associated with pathogenic B cells and/or T cells, particularly B cell and T cell-derived lymphoproliferative disorders or autoantibody-dependent autoimmune diseases.

This problem is solved by the features of the independent claims. Preferred embodiments of the present invention are provided by the dependent claims.

Therefore, the invention relates to a chimeric antigen receptor polypeptide (CAR), comprising:
i. an extracellular antigen-binding domain, comprising an antibody or antibody fragment that binds C-C chemokine receptor type 7 (CCR7) protein,
ii. a transmembrane domain, and
iii. an intracellular domain.

Therefore, the present invention relates to an anti-CCR7 CAR construct and corresponding immune cells expressing said construct, preferably a CAR-NK cell product that confers human NK cells with high cytotoxic activity against defined CCR7-positive cell lines, sparing healthy human cells and tissues.

In preferred embodiments of the immunotherapy approach of the present invention, patient-derived NK cells or allogeneic NK cells are transduced, preferably retrovirally, to express an artificial immune receptor as described herein, composed of an extracellular antibody-derived antigen recognition part, fused to a transmembrane section, and followed by intracellular signaling domains. The construct described herein, therefore, confers transduced NK cells with anti-tumor cytolytic capacity.

The anti-CCR7 CAR NK cell described herein is in preferred embodiments applicable to the treatment of patients suffering from any cell proliferative disorder characterized by pathogenic cells expressing CCR7. In embodiments, the patients are unable to be treated effectively with alternative approaches. More specifically, embodiments of the invention relate to the treatment of the following patient collectives:
i) patients with multidrug resistance,
ii) patients not eligible for allogeneic stem cell transplantation,
iii) patients with co-morbidities that preclude further chemotherapies,
iv) aged patients who do not tolerate chemotherapies,
v) the CAR is applicable for salvage therapies even after progressive disease and multiple lines of other standard-of-care therapies have failed,
vi) it is applicable even at low antigen density on target tumor cells, where antibodies can fail and/or
vii) it is applicable as a monotherapy, which is not the case for antibodies.

The anti-CCR7 CAR described herein confers high avidity to NK cells, necessary for anti-tumor efficacy. It has been demonstrated that the anti-CCR7 CAR of the present invention does not confer NK cell reactivity against physiological myeloid cell lineages, their precursors, and other non-hematopoietic tissue cells. Thus, the present invention has an unprecedentedly low off-target reactivity on myeloid hematopoietic and non-hematopoietic tissues. This is in contrast to anti-CD7 antibodies and/or anti-CD7 CAR constructs, another antigen used as a target for T cell malignancies, that is also expressed on a subset of common myeloid-T lymphoid progenitors.

By employing an anti-CCR7 CAR-NK cell product, fratricide can be reduced or circumvented, a hurdle that CAR-T cell approaches targeting T cell malignancies must overcome.

As demonstrated in the examples below, in an *in vitro* co-culture system, anti-CCR7 CAR-NK cells become activated upon exposure to CCR7-expressing human T-NHL tumor cell lines. These NK cells then develop an effector phenotype with high-level secretion of IFN-gamma, a phenotype predictive of cytotoxic activity.

Additionally, a cytotoxicity assay against selected target cell lines, such as T-NHL, B and T cell leukemia, CCR7-negative cells, and CCR7-transfectants shows that selective cellular cytotoxicity is obtained only in cell lines positive for CCR7.

Additional pre-clinical testing encompasses i) *in vitro* cytotoxicity testing against suitable T-NHL cell lines from patients and ii) *in vivo* testing of anti-CCR7 CAR activity against xenotransplanted T-NHL cell lines *in vivo.*

As such, the CAR of the present invention represents a surprising and beneficial approach toward treating the medical conditions described herein. The employment of anti-CCR7 CARs has not been previously attempted or described as a promising approach to treating NHL. The minimal (if not non-existent) unwanted side effects, due to the selectivity of the marker, also represent a beneficial and surprising aspect of the present invention.

In one embodiment, the invention relates to a chimeric antigen receptor (CAR) polypeptide as described herein, wherein said CAR is expressed in a genetically modified immune cell, preferably an NK cell, said immune cell binds CCR7 on the surface of a CCR7-expressing cell and is activated, thereby inducing cytotoxic activity against said CCR7-expressing cell.

Unlike most other lymphocytes, NK cells are controlled by multiple receptors that can react to infection or cellular transformation. One of the best-known mechanisms is the so-called missing-self theory (Kärre et al., Nature, 1986), where downregulation of MHC (major histocompatibility complex) class I heavy chain (self) molecules results in NK activation. This exemplifies the paradigmatic interlocking nature of the immune system, where inhibition of the adaptive arm of the immune response (antigen presentation by MHC to T cells) results in activation of the innate arm of the immune response (NK cells). Evasion of the immune system is a characteristic of both transformation and infection, so as both viral infections and tumors downregulate MHC molecules and block antigen presentation to T cells, both also activate NK cells.

It was entirely surprising that the balanced stimulation of the effector cells enabled the proliferation of said cells but had limited expression rates of CCR7.

Examples of CCR7-expressing cells are known to a skilled person and can be identified by further screening of cancers or other pathogenic cells. Cell lines expressing CCR7 are preferably DEL, DL-40, FE-DP, Mac-1, Mac-2a, HuT-78, Se-Ax, MyLa, HH, L-1236, L-591, SC-1, Raji, or JVM-3.

In one embodiment, the invention relates to a chimeric antigen receptor polypeptide (CAR), comprising:
- an extracellular antigen-binding domain comprising an antibody or antibody fragment that binds C-C chemokine receptor type 7 (CCR7) protein, wherein said antibody or antibody fragment comprises VH and VL domains of a single chain antibody fragment, wherein preferably a linker polypeptide is positioned between the VH and VL domains, wherein said linker is preferably configured to not interfere with the antibody fragment-CCR7 antigen interaction;
- a spacer polypeptide (also referred to as a hinge) positioned between the extracellular antigen-binding domain and a transmembrane domain, wherein said spacer polypeptide is preferably configured to not interfere with the antibody fragment-CCR7 antigen interaction and/or with NK cell activation when said CAR is expressed in an NK cell expressing said CAR;
- a transmembrane domain, wherein said transmembrane domain is preferably configured to not interfere with the antibody fragment-CCR7 antigen interaction and/or with NK cell activation when said CAR is expressed in an NK cell expressing said CAR;
- and an intracellular domain, wherein said intracellular (signaling) domain preferably comprises a co-stimulatory domain and an activation domain, wherein said intracellular domain is preferably configured to provide signals to stimulate NK cell activation upon binding to the CCR7 target, for example, by increasing cytokine production and/or facilitating NK cell replication, thus leading to a cytotoxic effect.

Therefore, the CAR of the present invention may employ various formats comprising potentially different protein sequences for each functional domain described herein. A skilled person is capable of selecting and testing the desired function of the CARs, for example, based on the experimental approaches demonstrated in the examples below. As such, the election of any given specific protein sequence to be used in the CAR of the invention in any of the functional domains discussed herein can be assessed by a skilled person using routine methods for functional efficacy. For example, various linker polypeptide sequences positioned between the VH and VL domains, various spacer polypeptide sequences (also referred to as a hinge) positioned between the extracellular antigen-binding domain and a transmembrane domain, various transmembrane domains, and various intracellular domains, preferably comprising co-stimulatory and signaling domains, may be employed.

In embodiments of the invention, the CAR and each of the elements or domains mentioned herein are configured to not detrimentally interfere with the antibody fragment-CCR7 antigen interaction, to not detrimentally interfere with NK cell activation when said CAR is expressed in an NK cell expressing said CAR, and to not detrimentally interfere with the CAR providing signals to stimulate NK cell activation upon binding to the CCR7 target.

Experimental approaches are described herein for assessing these properties of a CCR7 CAR, such that the invention is considered to encompass various functional sequence variants and combinations of domains of the types described herein without being limited to the particular sequences disclosed by way of example in the following. For example, specific activation of CAR-NK cells of the present invention by CCR7-expressing tumor cells can be demonstrated by the release of IFN-gamma or CD107a as shown below.

To the knowledge of the inventors, the present invention relates to the first described CCR7 CAR, and the first functional evidence of a desired therapeutic effect of a CCR7 CAR in a medical setting. Alone the provision of a CCR7 CAR, independent of the particular sequences employed in the various functional domains described herein, represents a significant and beneficial breakthrough in treating the many diseases associated with pathogenic mature B cells and/or memory B cells, and/or pathogenic T cells and/or T follicular helper cells.

### Embodiments relating to the antigen-binding domain of the CAR:

In one embodiment, the invention relates to a chimeric antigen receptor (CAR) polypeptide as described herein, wherein the antigen-binding domain comprises a variable heavy chain (VH), said VH comprising:
- a heavy chain complementary determining region 1 (H-CDR1) according to SEQ ID NO 1 (GFTFSNAA),
- a heavy chain complementary determining region 2 (H-CDR2) according to SEQ ID NO 2 (IRTKPNNFAT), and
- a heavy chain complementary determining region 3 (H-CDR3) according to SEQ ID NO 3 (TAGKDYFAY),
and a variable light chain (VL), said VL comprising:
- a light chain complementary determining region 1 (L-CDR1) according to SEQ ID NO 4 (KSLLFSDGKTY),
- a light chain complementary determining region 2 (L-CDR2) according to SEQ ID NO 5 (WMS), and
- a light chain complementary determining region 3 (L-CDR3) according to SEQ ID NO 6 (QQFLEFPFT).

In one embodiment, the invention relates to a chimeric antigen receptor (CAR) polypeptide as described herein, comprising a VH domain that comprises CDR sequences of SEQ ID NO. 1, SEQ ID No. 2 and SEQ ID NO. 3, and a VL domain that comprises CDR sequences of SEQ ID NO. 4; SEQ ID NO 5, and SEQ ID NO. 6.

In preferred embodiments, sequence variants with 80% or more sequence identity to the specific CDR sequences of SEQ ID 1-6 are encompassed in the invention. In embodiments, such sequence variants maintain CCR7 binding with essentially the same or similar functional properties as VH and VL domains with the specific CDR sequences of SEQ ID NO 1-6, i.e., the CCR7 binding is essentially the same or similar with respect to affinity, specificity and/or epitope binding mode.

The amino acid sequence of the scFV fragment was obtained originally from a rat anti-human CCR7 antibody and has been modified with respect to multiple improvements, for example by humanization of the VL and VH chains, in order to allow folding and expression in the context of a transmembrane receptor structure.

In some embodiments, the CDR sequences of the antigen binding domain may comprise 1 or 2 additional amino acids, in addition to those of SEQ ID NO 1-6, at N and/or C terminus end of the specific sequence of any one or more of SEQ ID NO 1-6. The additional amino acids at either one or more ends are, in embodiments, obtained from one or more of the VH and VL sequences presented herein. Thus, in embodiments, any one or more of the CDR sequences of the antigen binding domain may be 1 or 2 amino acids longer at one or both ends of the specific CDR sequences than those presented in SEQ ID NO 1-6, using additional sequences from the relevant VH and VL domains disclosed herein.

In some embodiments, the CDR sequences of the antigen binding domain may comprise 1 or 2 fewer amino acids, compared to those of SEQ ID NO 1-6, at N and/or C terminus ends of the specific sequence of any one or more of SEQ ID NO 1-6. Thus, in embodiments, any one or more of the six CDR sequences may be 1 or 2 amino acids shorter, at one or both ends, of the specific CDR sequences presented in SEQ ID NO 1-6.

Furthermore, the order of the light and heavy chain fragments may be inverted upon the desired configuration of the antigen-binding fragment.

Additionally, in some embodiments the linker sequence between heavy and light chains has been modified, for example by extending or shortening, in order to enhance the CAR function.

Additionally, the nucleic acid sequence encoding the CAR has been codon-optimized in order to improve expression of the CAR.

These modifications enable sufficient surface expression on NK cells and maintain proper antigen binding. High affinity and high avidity enable CAR-NK cells to i) recognize, ii) be activated against, and iii) kill tumor target cells with high, intermediate, or low CCR7 surface expression.

To the knowledge of the inventors, neither anti-CCR7 CARs nor humanized anti-CCR7 antibodies have been previously described in the art.

Surprisingly, the anti-CCR7 CAR NK cells are more efficient in recognizing the CCR7 antigen in the context of benign and transformed T cells and, to a much lesser extent, when CCR7 is expressed in benign and transformed B-cells. As seen in the examples, in primary blood lymphocytes, the antibody on which the CCR7 CAR of the present invention is based recognizes primarily CCR7 on T cells but not B cells. The preferential recognition of T cell-expressed CCR7 reduces the on-target off-tumor side effects on B cells. Small amounts of the CCR7 epitope, preferably 3D12, also appear to be present on B cells but significantly more on T cells and T cell neoplasia.

This property represents an unexpected and beneficial finding of the invention that could not have been predicted or derived from any given disclosure of the prior art. The CCR7 binding enabled for example by the CDR sequences disclosed herein, and their apparent preference towards binding CCR7-expressing T cells over B cells, enables reduced unwanted off target effects and thus is both unpredicted and beneficial, as no mention has been made previously of the particular antigen binding domain sequences disclosed herein and there is no earlier suggestion that these antigen-binding fragments may show preferential binding to T or B cells expressing the CCR7 target.

The anti-CCR7 CAR-NK cell product described herein is thus characterized by unique properties.

The anti-CCR7 CAR, as described herein, has a high affinity and confers high specificity and avidity to T or NK cells. These properties enable CAR-T or -NK cells to i) recognize, ii) be activated against, and iii) kill tumor target cells with high and intermediate CCR7 surface expression.

The number of CCR7 antigens expressed on the surfaces of tumor cells can be quantified by using an anti-CCR7 antibody coupled to a fluorescent dye in conjunction with Quantibrite beads (e.g. from Becton Dickinson). The preferred method to quantify CCR7 antigens expressed on the surfaces of tumor cells is "fluorescence-activated cell sorting/cell analysis" (FACS). The fluorescence intensity of beads correlates with the number of fluorescent antibodies bound to cells, which measures the number of CCR7 molecules on cells.

The VH and VL fragments described herein may be arranged in multiple configurations in the CAR and still maintain high specificity and high affinity for the target epitope. In some embodiments, the CAR may be configured in the VH-VL or VL-VH configuration, with variation in the linker, hinge, transmembrane domain, co-stimulatory domain, and/or activation domains, and still maintain its efficacy. This surprising feature of the invention enables greater flexibility in the design of CARs directed against CCR7, thereby enabling further modification and/or optimization of the CAR structure on the basis of the VH and VL domains described herein if any further development should be necessary or desired.

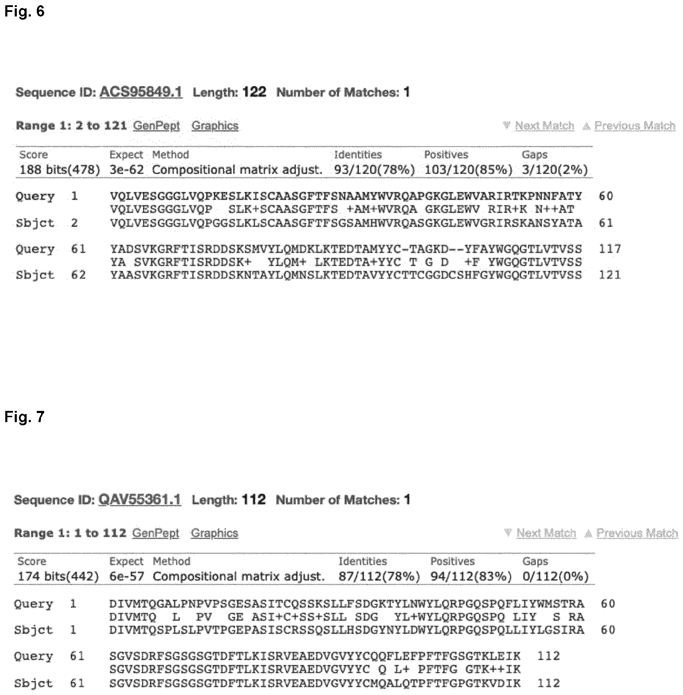

Sequence alignments of the rat and humanized sequences are shown in Fig. 6 and 7. Accordingly, the sequences below also encompass generalized sequences representing both rat and humanized forms of the antigen-binding domains. In the sequence below, each X represents a potential amino acid change. Preferred amino acid substitutions are described for each potentially altered position.

All possible combinations of potential modifications for any given potentially variant residue proposed herein (as identified by X in the "generalized sequences") are encompassed by the present invention. Combining one or more of these various substitutions may generate variants that exhibit the desired binding properties of the rat antigen-binding domain demonstrated herein. In some embodiments, these sequence variants are humanized variants of the original murine (rat) antibody. The CARs or parts thereof described herein also encompass a sequence with at least 70%, 80%, preferably 90%, more preferably 95% sequence identity, to those sequences disclosed explicitly or disclosed through a sequence formula.

In one embodiment, the invention relates to a chimeric antigen receptor (CAR) polypeptide as described herein, comprising a VH domain with at least 70%, 80% sequence identity, preferably at least 85%, 90%, 95% or with 100% sequence identity, to SEQ ID NO 7: 
X1VQLVESGGGLVQPX2X3SLKX4SCAASGFTFSX5X6AMX7WVRQAX8GKGLEWVX9RIR
X10KX11NX12X13ATX14YAX15SVKGRFTISRDDSKX16X17X18YLQMX19X20LKTEDTAX
21YYCTAGKDYFAYWGQGTLVTVSS,
 wherein X1: A or E; X2: G or K; X3: G or E; X4: L or I, X5: N orG; X6: A or S; X7: H or Y; X8: S or P; X9: G or A; X10: T or S; X11: P orA; X12: NorS;X13: F or Y; X14: Y or A; X15: D or A; X16: N or S; X17: M or T; X18: A or V; X19: N or D; X20: K or S; X21: V or M;
and a VL domain with at least 70% sequence identity, preferably at least 80%, 85%, 90%, or 95% sequence identity, to SEQ ID NO 8: 
DIVMTQX1X2LX3X4PVX5X6GEX7ASIX8CX9SSX10SLLX11SDGX12TYLX13WYLQX14PG
QSPQX15LIYX16X17SX18RASGVX19DRFSGSGSGTDFTLKISRVEAEDVGVYYCX20QX2
1X22EFPFTFGSGTKLEIKR
 wherein X1: T or G; X2: P orA; X3: S or P; X4: L or N; X5: T or P; X6: P or S; X7: P or S; X8: S or T; X9: RorQ;X10: KorQ;X11: F or D; X12: KorN;X13: D or N; X14: KorR;X15: L or F; X16: WorT;X17: M or L; X18: Y or T; X19: P or S; X20: Q or M; X21: For R; X22: L or I.

The above-sequence formulae relate to VH and VL sequences of the antigen-binding domain of the CAR, with variation at particular positions of the sequence, as tested by the inventors to show suitable binding characteristics. In embodiments, any of the "X"-positions disclosed above may be replaced by any given amino acid, preferably by the particular amino acids disclosed above. Variation in sequence at any one or more of the "X"-positions is encompassed in the invention. Any combination of amino acid variants, disclosed at any one or more of the variable positions, is encompassed in the present invention.

In embodiments, the percentage sequence identity relates to the full length of variable VH and VL domains provided herein.

In preferred embodiments, the sequence variants with 80% or more sequence identity to the specific VH and VL sequences listed herein maintain CCR7 binding with essentially the same or similar functional properties as VH and VL domains with the specific sequences recited herein, i.e., the CCR7 binding is essentially the same or similar with respect to affinity, specificity, and epitope binding mode.

In one embodiment, the invention relates to a chimeric antigen receptor (CAR) polypeptide as described herein, comprising a VH domain according to
SEQ ID NO 9 
(EVQLVESGGGLVQPGGSLKLSCAASGFTFSNAAMHWVRQASGKGLEWVGRIRTKPNNFA
TAYAASVKGRFTISRDDSKNTAYLQMNKLKTEDTAVYYCTAGKDYFAYWGQGTLVTVSS),
 or
SEQ ID NO 10 
(EVQLVESGGGLVQPKESLKLSCAASGFTFSNAAMYWVRQAPGKGLEWVARIRTKPNNFAT
YYADSVKGRFTISRDDSKSMAYLQMNKLKTEDTAMYYCTAGKDYFAYWGQGTLVTVSS),
 or
SEQ ID NO 11 
(EVQLVESGGGLVQPGGSLKLSCAASGFTFSNAAMYWVRQAPGKGLEWVARIRTKPNNFA
TYYADSVKGRFTISRDDSKSMAYLQMNKLKTEDTAMYYCTAGKDYFAYWGQGTLVTVSS),
 or
SEQ ID NO 12 
(EVQLVESGGGLVQPKESLKLSCAASGFTFSNAAMHWVRQASGKGLEWVGRIRTKPNNFA
TYYADSVKGRFTISRDDSKSMAYLQMNKLKTEDTAMYYCTAGKDYFAYWGQGTLVTVSS),
 or
SEQ ID NO 13 
(EVQLVESGGGLVQPKESLKLSCAASGFTFSNAAMYWVRQAPGKGLEWVARIRTKPNNFAT
AYAASVKGRFTISRDDSKSMAYLQMNKLKTEDTAMYYCTAGKDYFAYWGQGTLVTVSS),
 or
SEQ ID NO 14 
(EVQLVESGGGLVQPKESLKLSCAASGFTFSNAAMYWVRQAPGKGLEWVARIRTKPNNFAT
YYADSVKGRFTISRDDSKNTAYLQMNKLKTEDTAVYYCTAGKDYFAYWGQGTLVTVSS),
 or
SEQ ID NO 15 
(EVQLVESGGGLVQPGGSLKLSCAASGFTFSGSAMHWVRQASGKGLEWVGRIRSKANSYA
TAYAASVKGRFTISRDDSKNTAYLQMNSLKTEDTAVYYCTAGKDYFAYWGQGTLVTVSS),
 or
SEQ ID NO 16 
(AVQLVESGGGLVQPKESLKISCAASGFTFSNAAMYWVRQAPGKGLEWVARIRTKPNNFAT
YYADSVKGRFTISRDDSKSMVYLQMDKLKTEDTAMYYCTAGKDYFAYWGQGTLVTVSS),
 and a VL domain according to
SEQ ID NO 17 
(DIVMTQTPLSLPVTPGEPASISCRSSKSLLFSDGKTYLDWYLQKPGQSPQLLIYWMSYRAS
GVPDRFSGSGSGTDFTLKISRVEAEDVGVYYCQQFLEFPFTFGSGTKLEIKR),
 or
SEQ ID NO 18 
(DIVMTQTPLSLPVTPGEPASISCRSSKSLLFSDGKTYLNWYLQKPGQSPQLLIYWMSYRAS
GVPDRFSGSGSGTDFTLKISRVEAEDVGVYYCQQFLEFPFTFGSGTKLEIKR)
SEQ ID NO 19 
(DIVMTQGALPNPVTSGESASISCQSSKSLLFSDGKTYLDWYLQKPGQSPQFLIYWMSTRAS
GVSDRFSGSGSGTDFTLKISRVEAEDVGVYYCQQFLEFPFTFGSGTKLEIKR),
 or
SEQ ID NO20 
(DIVMTQGALPNPVTSGESASISCQSSKSLLFSDGKTYLNWYLQKPGQSPQFLIYWMSTRAS
GVSDRFSGSGSGTDFTLKISRVEAEDVGVYYCQQFLEFPFTFGSGTKLEIKR)
SEQ ID NO 21 
(DIVMTQTPLSNPVTSGESASISCQSSKSLLFSDGKTYLNWYLQKPGQSPQFLIYWMSTRAS
GVSDRFSGSGSGTDFTLKISRVEAEDVGVYYCQQFLEFPFTFGSGTKLEIKR),
 or
SEQ ID NO 22 
(DIVMTQGALPLPVTPGESASISCQSSKSLLFSDGKTYLNWYLQKPGQSPQFLIYWMSTRAS
GVSDRFSGSGSGTDFTLKISRVEAEDVGVYYCQQFLEFPFTFGSGTKLEIKR),
 or
SEQ ID NO 23 
(DIVMTQGALPNPVTSGEPASISCRSSKSLLFSDGKTYLNWYLQKPGQSPQFLIYWMSTRAS
GVSDRFSGSGSGTDFTLKISRVEAEDVGVYYCQQFLEFPFTFGSGTKLEIKR),
 or
SEQ ID NO 24 
(DIVMTQGALPNPVTSGESASISCQSSKSLLFSDGKTYLNWYLQKPGQSPQLLIYWMSYRAS
GVPDRFSGSGSGTDFTLKISRVEAEDVGVYYCQQFLEFPFTFGSGTKLEIKR),
 or
SEQ ID NO 25 
(DIVMTQTPLSLPVTPGEPASISCRSSKSLLFSDGKTYLNWYLQKPGQSPQLLIYWMSYRAS
GVPDRFSGSGSGTDFTLKISRVEAEDVGVYYCQQFLEFPFTFGSGTKLEIKR),
 or
SEQ ID NO 26 
(DIVMTQTPLSLPVTPGEPASISCRSSQSLLDSDDGNTYLDWYLQKPGQSPQLLIYTLSYRAS
GVPDRFSGSGSGTDFTLKISRVEAEDVGVYYCMQRIEFPFTFGSGTKLEIKR),
 or
SEQ ID NO 27

(DIVMTQGALPNPVPSGESASITCQSSKSLLFSDGKTYLNWYLQRPGQSPQFLIYWMSTRAS
GVSDRFSGSGSGTDFTLKISRVEAEDVGVYYCQQFLEFPFTFGSGTKLEIKR).

In embodiments, the invention relates to a CAR comprising an antigen binding domain with VH and VL sequences with at least 70, 80, 90 or 95% sequence identity to one of the specific VH and VL sequences disclosed herein (preferably SEQ ID NO 9-16 for the VH and SEQ ID NO 17-27 for the VL). In embodiments, any one of SEQ ID NO 9-16 for the VH and any one of SEQ ID NO 17-27 for the VL may be combined, in any combination, whilst achieving functional and beneficial properties as described herein.

In embodiments, the invention relates to a CAR comprising an antigen binding domain with VH and VL sequences with at least 70, 80, 90 or 95% sequence identity to one of the specific VH and VL sequences disclosed herein (preferably SEQ ID NO 9-16 for the VH and SEQ ID NO 17-27 for the VL), also comprising the CDR sequences according to SEQ ID NO 1-6.

In embodiments, the CDR sequences may therefore be used to define the antigen binding domain of the CAR of the present invention, and the VH and VL domains may, in the flanking framework regions exhibit some sequence variation over the specific sequences disclosed herein. In embodiments, the CDR sequences may, on their own, be sufficient to define the inventive CAR, without reference to specific framework sequences.

A further aspect of the present invention relates to an antibody, or an antigen-binding fragment of an antibody, based on the antigen-binding domain of the CAR disclosed herein.

In embodiments, the antibody or antigen-binding fragment of the antibody comprises one or more of the CDR and/or VH and/or VL sequences of the antigen-binding domain disclosed herein. As disclosed with respect to the CAR of the present invention, the antigen-binding domain of the inventive CAR shows a preference to bind CCR7 presented on T cells compared to CCR7 presented on B cells, thus providing an unexpected beneficial property that could not have been derived from the prior art.

This property relates to the inventive CAR, to the antibody itself, from which the antigen-binding domain of the CAR is derived, and to the antigen-binding domain of the CAR itself, and to the antigen-binding fragment of a corresponding antibody of the present invention. The sequences disclosed herein with respect to the antigen-binding domain of the inventive CAR are also considered to be disclosed in the context of the inventive antibody, or the antigen-binding fragment of the antibody.

### Embodiments relating to further components of the CAR:

In further embodiments, the invention relates to a chimeric antigen receptor (CAR) polypeptide that comprises one or more linkers, spacers, transmembranes, and signaling domains.

In one embodiment, the CAR comprises an intracellular domain comprising a co-stimulatory domain and a signaling (activation) domain.

In one embodiment, the invention relates to a chimeric antigen receptor (CAR) polypeptide as described herein, wherein the extracellular antigen-binding domain comprises a linker polypeptide positioned between the VH and VL domains, wherein said linker is preferably selected from:
- a Whitlow (SEQ ID NO 28; GSTSGSGKPGSGEGSTKG), or
- Gly-Ser (SEQ ID NO 35; SSGGGGSGGGGSGGGGS) linker, or
- linkers with at least 80% sequence identity to SEQ ID NO 28 or 35.

In preferred embodiments, the linker is a Whitlow linker.

In one embodiment, the invention relates to a chimeric antigen receptor (CAR) polypeptide as described herein, comprising additionally a spacer polypeptide positioned between the extracellular antigen-binding domain and the transmembrane domain, wherein said spacer is selected from:
- IgG1 spacer (SEQ ID NO 29; 
PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGKKDPK),
- IgG1Δ spacer (SEQ ID NO 36; 
PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSSLSPGKK),
- IgG4 (Hi-CH2-CH3) spacer (SEQ ID NO 37; 
ESKYGPPCPPCPAPEFEGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK),
- IgG4 (Hi-CH3) spacer (SEQ ID NO 38; 
ESKYGPPCPPCPGQPREPQVYTLPPSQEEMTKNQVSLTCLVKGFYPSDIAVEWESNGQPE
NNYKTTPPVLDSDGSFFLYSRLTVDKSRWQEGNVFSCSVMHEALHNHYTQKSLSLSLGK),
- IgG4 (Hi) spacer (SEQ ID NO 39; ESKYGPPCPPCP), or
- a spacer with at least 80% sequence identity to any one of SEQ ID NO 29, 36 to 39.

In preferred embodiments, the spacer is a IgG1 spacer.

In one embodiment, the invention relates to a chimeric antigen receptor (CAR) polypeptide as described herein, wherein the transmembrane domain is selected from:
- a CD28 domain (SEQ ID NO 30; FWVLVVVGVLACYSLLVTVAFIIFWV), or
- a CD8a domain (SEQ ID NO 40; IYIWAPLAGTCGVLLLSLVITLYC), or
- transmembrane domains with at least 80% sequence identity to SEQ ID NO 30 or 40.

In preferred embodiments, the transmembrane domain is a CD28 domain.

In one embodiment, the invention relates to a chimeric antigen receptor (CAR) polypeptide as described herein, wherein the intracellular domain comprises:
- a CD28 co-stimulatory domain (SEQ ID NO 31;
   RSKRSRLLHSDYMNMTPRRPGPTRKHYQPYAPPRDFAAYRSL), and/or
- a co-stimulatory domain selected from a 4-1BB co-stimulatory domain (SEQ ID NO 41; KRGRKKLLYIFKQPFMRPVQTTQEEDGCSCRFPEEEEGGCEL), and/or
- a co-stimulatory domain comprising both a 4-1BB (SEQ ID NO 41) and a CD28 co-stimulatory domain (SEQ ID NO 31) arranged adjacently, or
- a co-stimulatory domain with at least 80% sequence identity to SEQ ID NO 31 or 41.

In preferred embodiments, the intracellular domain comprises a CD28 co-stimulatory domain. In contrast to CARs based on the 4-1BB co-stimulatory domain, those employing CD28 demonstrate a more rapid effector profile. This heightened efficiency is associated with substantial alterations in lymphocyte-specific protein tyrosine kinase (Lck) phosphorylation within the signaling pathway. Consequently, CD28-triggered signals elicit increased levels of key immune effectors, including interferon-γ (IFN-γ), granzyme B, and tumor necrosis factor α (TNF-α). This differential signaling pathway activation suggests that CD28-based CARs may contribute to enhanced cytotoxicity and immune response.

In one embodiment, the invention relates to a chimeric antigen receptor (CAR) polypeptide as described herein, comprising additionally a signaling domain (otherwise known as an activation domain), wherein said signaling domain is:
- a CD3zeta (4-1BB or CD28) signaling domain (SEQ ID NO 32; 
LRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR),
 or
- a signaling domain with at least 80% sequence identity to SEQ ID NO 32.

In preferred embodiments, the invention relates to a chimeric antigen receptor (CAR) polypeptide as described herein:
- wherein the extracellular antigen-binding domain comprises a linker polypeptide positioned between the VH and VL domains, wherein said linker is preferably a Whitlow linker (SEQ ID NO 28; GSTSGSGKPGSGEGSTKG) or linkers with at least 80% sequence identity to SEQ ID NO 28; and/or
- comprising additionally a spacer polypeptide positioned between the extracellular antigen-binding domain and the transmembrane domain, wherein said spacer is preferably an IgG1 spacer (SEQ ID NO 29; PAEPKSPDKTHTCPPCPAPPVAGPSVFLFPPKPKDTLMIARTPEVTCVVVDVSHED PEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNH YTQKSLSLSPGKKDPK),
   or a spacer with at least 80% sequence identity to SEQ ID
   NO 29; and/or
- wherein the transmembrane domain is a CD28 domain (SEQ ID NO 30; FWVLVVVGGVLACYSLLVTVAFIIFWV), or transmembrane domain with at least 80% sequence identity to SEQ ID NO 30; and/or
- wherein the intracellular domain comprises a CD28 co-stimulatory domain (SEQ ID NO 31; RSKRSRLLHSDYMNMTPRRPGPTRKHYQPYAPPRDFAAYRSL), or a co-stimulatory domain with at least 80% sequence identity to SEQ ID NO 31; and/or
- comprising additionally a signaling domain (activation domain), wherein said signaling domain is preferably a CD3zeta signaling domain (SEQ ID NO 32; LRVKFSRSADAPAYQQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR) or a signaling domain with at least 80% sequence identity to SEQ ID NO 32; and/or
- comprising additionally a signal peptide, wherein said signal peptide is preferably an IgK signal peptide (SEQ ID NO. 33; MDFQVQIFSFLLISASVIMSR) or a signal peptide with at least 80% sequence identity to SEQ ID NO 33.

In preferred embodiments, the chimeric antigen receptor (CAR) polypeptide described herein comprises or consists of a sequence according to SEQ ID NO 34.

As demonstrated herein, the various signaling domains may be exchanged in multiple configurations, providing a CAR with flexibility with respect to its design without losing advantageous binding properties.

Due to the variants (by adding alternative components) employed as the linker, spacer, transmembrane, and intracellular domains, it becomes apparent that the various components may be exchanged as required by the skilled person. The CCR7 binding properties may be maintained, thereby maintaining the desired biological effects.

In preferred embodiments, in combination with the MP71-vector and a gamma-retrovirus expression system, an unusually high transduction rate for human NK cells can be achieved. The transduction system is variable due to a modular design of the CAR construct, meaning that lentiviruses as well as transposons can be employed, depending on the needs and preferences of the skilled person when carrying out the invention.

Transfer of the genetic information/nucleic acid molecule for the CCR7 CAR also includes CRISPR/Cas and TALEN-mediated insertion into target cell lines, preferably Natural Killer Cells, T lymphocytes, and induced pluripotent stem cells (iPSCs). All suitable methods for transferring the genetic information/nucleic acid molecule for the CCR7 CAR into the cell expressing said CAR are encompassed by the present invention, and a suitable method may be selected by a skilled person when carrying out the invention. For example, multiple methods of transforming NK cells are known in the art, including any given viral-based gene transfer method, such as those based on modified Lentiviridae, and non-viral methods such as DNA-based transposons and direct transfer of mRNA by electroporation.

Additionally, the signaling components of the CAR construct have been exchanged in a three step cloning procedure that allows for a modular composition, and tailor-made construction by a skilled person, of clinically applicable anti-CCR7 CARs.

Primary human NK cells from various sources and a highly efficient NK cell production process featuring unusually high retroviral transduction rates have been developed for proof-of-concept analysis. In addition, the NK cell line NK-92 for transduction and functional assays was employed, demonstrating cytokine secretion and cytolytic activity when equipped with the anti-CCR7 CAR.

In one embodiment, the NK cell of said CCR7 CAR construct is derived from NK-92 cells. NK-92, the first NK cell-based immunotherapy to receive Investigational New Drug approval by the US Food and Drug Administration (FDA) for clinical testing, is a homogeneous, immortalized NK lymphoma cell line that can be expanded *ex vivo* to achieve large cell numbers38. NK-92 cells lack expression of most killer cell immunoglobulin-like receptors (KIRs) and are thus less likely to become inhibited (Laskowski T et a., Nat Rev Cancer, 2022).

### Further aspects of the invention:

In a further aspect of the invention, the invention relates to an isolated nucleic acid molecule encoding the CAR of the invention, encoding an antigen-binding domain of the CAR, encoding the antibody of the invention, or encoding the antigen-binding fragment of the antibody of the present invention.

In a further aspect of the invention, the invention relates to an isolated nucleic acid molecule, preferably in the form of a vector, such as a retroviral vector, lentiviral vector, or transposon, encoding a chimeric antigen receptor (CAR) polypeptide, selected from the group consisting of:
a) a nucleic acid molecule comprising a nucleotide sequence:
   - which encodes a chimeric antigen receptor (CAR) polypeptide according to any embodiment of the CAR described herein,
   - which encodes an extracellular antigen-binding domain, a transmembrane domain, and an intracellular domain, wherein the extracellular antigen-binding domain is encoded by at least one sequence of SEQ ID NO 34,
   - according to SEQ ID No 34, and/or
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising preferably a sequence identity to a nucleotide sequence according to a) or b) of at least 80%;
d) a nucleic acid molecule which, as a consequence of the genetic code, is degenerate to a nucleotide sequence according to a) through c); and/or
e) a nucleic acid molecule according to a nucleotide sequence of a) through d) which is modified by deletions, additions, substitutions, translocations, inversions and/or insertions and is functionally analogous/equivalent to a nucleotide sequence according to a) through d).

The term degenerate to (or degenerated into) refers to differences in nucleotide sequence of a nucleic acid molecule, but according to the genetic code, do not lead to differences in amino acid protein product of the nucleotide sequence after translation. In one embodiment, the nucleic acid molecule relates to the above molecules under a) and b), a), b) and c), or a), b) and d).

**Table 1. Preferred amino acid and nucleotide sequences of the present invention:**

| **SEQ ID No.** | **Sequence** | **Description** |
|---|---|---|
| 1 | GFTFSNAA | H-CDR1 |
| 2 | IRTKPNNFAT | H-CDR2 |
| 3 | TAGKDYFAY | H-CDR3 |
| 4 | KSLLFSDGKTY | L-CDR1 |
| 5 | WMS | L-CDR2 |
| 6 | QQFLEFPFT | L-CDR3 |
| 7 | X1VQLVESGGGLVQPX2X3SLKX4SCAASGFTFSX5X6AMX7WVRQAX8GK GLEWVX9RIRX10KX11NX12X13ATX14YAX15SVKGRFTISRDDSKX16 X17X18YLQMX19X20LKTEDTAX21YYCTAGKDYFAYWGQGTLVTVSS, wherein X1 to X21 can be any amino acid, preferably wherein X1: A or E; X2: G or K; X3: G or E; X4: L or I, X5: N or G; X6: A or S; X7: H or Y; X8: S or P; X9: G or A; X10: T or S; X11: P or A; X12: N or S; X13: F or Y; X14: Y or A; X15: D or A; X16: N or S; X17: M or T; X18: A or V; X19: N or D; X20: K or S; X21: V or M; | VH sequence "generalized" encompassing both rat and humanized sequences |
| 8 | DIVMTQX1X2LX3X4PVX5X6GEX7ASIX8CX9SSX10SLLX11SDGX12T YLX13WYLQX14PGQSPQX15LIYX16X17SX18RASGVX19DRFSGSGSG TDFTLKISRVEAEDVGVYYCX20QX21X22EFPFTFGSGTKLEIKR, wherein X1 to X22 can be any amino acid, preferably wherein X1: T or G; X2: P or A; X3: S or P; X4: L or N; X5: T or P; X6: P or S; X7: P or S; X8: S or T; X9: R or Q; X10: K or Q; X11: F or D; X12: K or N; X13: D or N; X14: K or R; X15: L or F; X16: W or T; X17: M or L; X18: Y or T; X19: P or S; X20: Q or M; X21: F or R; X22: L or I. | VL sequence "generalized" encompassing both rat and humanized sequences |
| 9 | EVQLVESGGGLVQPGGSLKLSCAASGFTFSNAAMHWVRQASGKGLEWVGRI RTKPNNFATAYAASVKGRFTISRDDSKNTAYLQMNKLKTEDTAVYYCTAGK DYFAYWGQGTLVTVSS | Fully humanized (fh) VH |
| 10 | EVQLVESGGGLVQPKESLKLSCAASGFTFSNAAMYWVRQAPGKGLEWVARI RTKPNNFATYYADSVKGRFTISRDDSKSMAYLQMNKLKTEDTAMYYCTAGK DYFAYWGQGTLVTVSS | Partially humanized (ph) VH-1 |
| 11 | EVQLVESGGGLVQPGGSLKLSCAASGFTFSNAAMYWVRQAPGKGLEWVARI RTKPNNFATYYADSVKGRFTISRDDSKSMAYLQMNKLKTEDTAMYYCTAGK DYFAYWGQGTLVTVSS | Partially humanized (ph) VH-2 |
| 12 | EVQLVESGGGLVQPKESLKLSCAASGFTFSNAAMHWVRQASGKGLEWVGRI RTKPNNFATYYADSVKGRFTISRDDSKSMAYLQMNKLKTEDTAMYYCTAGK DYFAYWGQGTLVTVSS | Partially humanized (ph) VH-3 |
| 13 | EVQLVESGGGLVQPKESLKLSCAASGFTFSNAAMYWVRQAPGKGLEWVARI RTKPNNFATAYAASVKGRFTISRDDSKSMAYLQMNKLKTEDTAMYYCTAGK DYFAYWGQGTLVTVSS | Partially humanized (ph) VH-4 |
| 14 | EVQLVESGGGLVQPKESLKLSCAASGFTFSNAAMYWVRQAPGKGLEWVARI RTKPNNFATYYADSVKGRFTISRDDSKNTAYLQMNKLKTEDTAVYYCTAGK DYFAYWGQGTLVTVSS | Partially humanized (ph) VH-5 |
| 15 | EVQLVESGGGLVQPGGSLKLSCAASGFTFSGSAMHWVRQASGKGLEWVGRI RSKANSYATAYAASVKGRFTISRDDSKNTAYLQMNSLKTEDTAVYYCTAGK DYFAYWGQGTLVTVSS | Human VH |
| 16 | VQLVESGGGLVQPKESLKISCAASGFTFSNAAMYWVRQAPGKGLEWVARIR TKPNNFATYYADSVKGRFTISRDDSKSMVYLQMDKLKTEDTAMYYCTAGKD YFAYWGQGTLVTVSS | Rat VH |
| 17 | DIVMTQTPLSLPVTPGEPASISCRSSKSLLFSDGKTYLDWYLQKPGQSPQL LIYWMSYRASGVPDRFSGSGSGTDFTLKISRVEAEDVGVYYCQQFLEFPFT FGSGTKLEIKR | Fully humanized (fh) VL-1 |
| 18 | DIVMTQTPLSLPVTPGEPASISCRSSKSLLFSDGKTYLNWYLQKPGQSPQL LIYWMSYRASGVPDRFSGSGSGTDFTLKISRVEAEDVGVYYCQQFLEFPFT FGSGTKLEIKR | Fully humanized (fh) VL-2 |
| 19 | DIVMTQGALPNPVTSGESASISCQSSKSLLFSDGKTYLDWYLQKPGQSPQF LIYWMSTRASGVSDRFSGSGSGTDFTLKISRVEAEDVGVYYCQQFLEFPFT FGSGTKLEIKR | Partially humanized (ph) VL-1 |
| 20 | DIVMTQGALPNPVTSGESASISCQSSKSLLFSDGKTYLNWYLQKPGQSPQF LIYWMSTRASGVSDRFSGSGSGTDFTLKISRVEAEDVGVYYCQQFLEFPFT FGSGTKLEIKR | Partially humanized (ph) VL-2 |
| 21 | DIVMTQTPLSNPVTSGESASISCQSSKSLLFSDGKTYLNWYLQKPGQSPQF LIYWMSTRASGVSDRFSGSGSGTDFTLKISRVEAEDVGVYYCQQFLEFPFT FGSGTKLEIKR | Partially humanized (ph) VL-3 |
| 22 | DIVMTQGALPLPVTPGESASISCQSSKSLLFSDGKTYLNWYLQKPGQSPQF LIYWMSTRASGVSDRFSGSGSGTDFTLKISRVEAEDVGVYYCQQFLEFPFT FGSGTKLEIKR | Partially humanized (ph) VL-4 |
| 23 | DIVMTQGALPNPVTSGEPASISCRSSKSLLFSDGKTYLNWYLQKPGQSPQF LIYWMSTRASGVSDRFSGSGSGTDFTLKISRVEAEDVGVYYCQQFLEFPFT FGSGTKLEIKR | Partially humanized (ph) VL-5 |
| 24 | DIVMTQGALPNPVTSGESASISCQSSKSLLFSDGKTYLNWYLQKPGQSPQL LIYWMSYRASGVPDRFSGSGSGTDFTLKISRVEAEDVGVYYCQQFLEFPFT FGSGTKLEIKR | Partially humanized (ph) VL-6 |
| 25 | DIVMTQTPLSLPVTPGEPASISCRSSKSLLFSDGKTYLNWYLQKPGQSPQL LIYWMSYRASGVPDRFSGSGSGTDFTLKISRVEAEDVGVYYCQQFLEFPFT FGSGTKLEIKR | Partially humanized (ph) VL-7 |
| 26 | DIVMTQTPLSLPVTPGEPASISCRSSQSLLDSDDGNTYLDWYLQKPGQSPQ LLIYTLSYRASGVPDRFSGSGSGTDFTLKISRVEAEDVGVYYCMQRIEFPF TFGSGTKLEIKR | Human VL |
| 27 | DIVMTQGALPNPVPSGESASITCQSSKSLLFSDGKTYLNWYLQRPGQSPQF LIYWMSTRASGVSDRFSGSGSGTDFTLKISRVEAEDVGVYYCQQFLEFPFT FGSGTKLEIK | Rat VL |
| 28 | GSTSGSGKPGSGEGSTKG | Whitlow |
| 29 | PAEPKSPDKTHTCPPCPAPPVAGPSVFLFPPKPKDTLMIARTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTC | IgG1 spacer |
| | LVKGFYPSDIAVEWESNGQPENNYKTTPPVLDSDGSFFLYSKLTVDKSRWQ QGNVFSCSVMHEALHNHYTQKSLSLSPGKKDPK | |
| 30 | FWVLVVVGGVLACYSLLVTVAFIIFWV | Transmembra ne domain (CD28) |
| 31 | RSKRSRLLHSDYMNMTPRRPGPTRKHYQPYAPPRDFAAYRSL | Co-stimulatory domain (CD28) |
| 32 | LRVKFSRSADAPAYQQGQNQLYNELNLGRREEYDVLDKRRGRDPEMGGKPR RKNPQEGLYNELQKDKMAEAYSEIGMKGERRRGKGHDGLYQGLSTATKDTY DALHMQALPPR | CD3zeta signaling domain |
| 33 | MDFQVQIFS FLLISASVIMSR | IgK signal peptide |
| 34 | MDFQVQIFSFLLISASVIMSRAVQLVESGGGLVQPKESLKISCAASGFTFS NAAMYWVRQAPGKGLEWVARIRTKPNNFATYYADSVKGRFTISRDDSKSMV YLQMDKLKTEDTAMYYCTAGKDYFAYWGQGTLVTVSSGSTSGSGKPGSGEG STKGDIVMTQGALPNPVPSGESASITCQSSKSLLFSDGKTYLNWYLQRPGQ SPQFLIYWMSTRASGVSDRFSGSGSGTDFTLKISRVEAEDVGVYYCQQFLE FPFTFGSGTKLEIKRPAEPKSPDKTHTCPPCPAPPVAGPSVFLFPPKPKDT LMIARTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGS FFLYSKLTVDKSRWQQGNVFSCSVMHEALHNHYTQKSLSLSPGKKDPKFWV LVVVGGVLACYSLLVTVAFIIFWVRSKRSRLLHSDYMNMTPRRPGPTRKHY QPYAPPRDFAAYRSLRVKFSRSADAPAYQQGQNQLYNELNLGRREEYDVLD KRRGRDPEMGGKPRRKNPQEGLYNELQKDKMAEAYSEIGMKGERRRGKGHD GLYQGLSTATKDTYDALHMQALPPR | Non-humanized anti-CCR7 CAR construct |
| 35 | SSGGGGSGGGGSGGGGS | Gly-Ser linker |
| 36 | PAEPKSPDKTHTCPPCPAPPVAGPSVFLFPPKPKDTLMIARTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSRWQ QGNVFSCSVMHEALHNHYTQKSLSSLSPGKK | IgG1Δ spacer |
| 37 | ESKYGPPCPPCPAPEFEGGPSVFLFPPKPKDTLMISRTPEVTCVVVDVSQE DPEVQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGNV FSCSVMHEALHNHYTQKSLSLSLGK | IgG4 (Hi-CH2-CH3) spacer |
| 38 | ESKYGPPCPPCPGQPREPQVYTLPPSQEEMTKNQVSLTCLVKGFYPSDIAV EWESNGQPENNYKTTPPVLDSDGSFFLYSRLTVDKSRWQEGNVFSCSVMHE ALHNHYTQKSLSLSLG | IgG4 (Hi-CH3) spacer |
| 39 | ESKYGPPCPPCP | IgG4 (Hi) spacer |
| 40 | IYIWAPLAGTCGVLLLSLVITLYC | CD8a domain |
| 41 | KRGRKKLLYIFKQPFMRPVQTTQEEDGCSCRFPEEEEGGCEL | 4-1 BB co-stimulatory domain |
| 42 | ATGGATTTTCAGGTGCAGATCTTCAGCTTCCTGCTGATCTCCGCCAGCGTG ATCATGAGCAGAGCCGTGCAGCTGGTGGAATCTGGCGGAGGACTGGTGCAG CCTAAAGAGAGCCTGAAGATCAGCTGTGCCGCCAGCGGCTTCACCTTTAGC AATGCCGCCATGTACTGGGTCCGACAGGCCCCTGGAAAAGGCCTGGAATGG GTTGCCCGGATCAGGACCAAGCCTAACAACTTCGCCACCTACTACGCCGAC AGCGTGAAGGGCAGATTCACCATCAGCCGGGACGACAGCAAGAGCATGGTG TACCTGCAGATGGACAAGCTGAAAACCGAGGACACCGCTATGTACTACTGC ACCGCCGGCAAGGACTACTTCGCCTATTGGGGACAGGGCACCCTGGTCACA GTGTCTAGCGGCTCTACAAGCGGCTCTGGCAAACCTGGATCTGGCGAGGGA AGCACCAAGGGCGATATCGTGATGACACAGGGCGCTCTGCCCAATCCTGTG CCTTCTGGCGAGAGCGCCTCTATCACCTGTCAGAGCAGCAAGTCCCTGCTG TTCAGCGACGGCAAGACCTACCTGAACTGGTATCTGCAGAGGCCCGGACAG AGCCCTCAGTTCCTGATCTACTGGATGAGCACCAGAGCCAGCGGCGTGTCC GATAGATTTTCTGGCAGCGGCTCCGGCACCGACTTCACCCTGAAGATTAGC AGAGTGGAAGCCGAGGACGTGGGCGTGTACTATTGCCAGCAGTTCCTGGAA TTCCCATTCACCTTCGGCAGCGGCACCAAGCTGGAAATCAAGAGACCCGCC GAGCCCAAGAGCCCCGACAAGACCCATACCTGCCCTCCATGTCCTGCCCCT CCAGTGGCTGGCCCTAGCGTGTTCCTGTTCCCCCCAAAGCCCAAGGACACC CTGATGATCGCCCGGACCCCTGAAGTGACCTGCGTGGTGGTGGATGTGTCC CACGAGGATCCCGAAGTGAAGTTCAATTGGTACGTGGACGGCGTGGAAGTG CACAACGCCAAGACCAAGCCCAGAGAGGAACAGTACAACAGCACCTACCGG GTGGTGTCTGTGCTGACCGTGCTGCATCAGGACTGGCTGAACGGCAAAGAG TACAAGTGCAAGGTGTCCAACAAGGCCCTGCCTGCCCCCATCGAGAAAACC ATCTCCAAGGCCAAGGGACAGCCCCGCGAGCCCCAGGTGTACACACTGCCT CCAAGCAGGGACGAGCTGACCAAGAACCAGGTCAGCCTGACCTGCCTGGTC AAAGGCTTCTATCCCAGCGACATCGCCGTGGAGTGGGAGAGCAATGGGCAG CCGGAGAACAACTACAAGACCACGCCTCCCGTGCTGGACTCCGACGGCTCC TTCTTCCTCTACAGCAAGCTCACCGTGGACAAGAGCAGGTGGCAGCAGGGG AACGTCTTCTCATGCTCCGTGATGCATGAGGCTCTGCACAACCACTACACG CAGAAGAGCCTCTCCCTGTCTCCGGGTAAAAAAGATCCCAAATTTTGGGTG CTGGTGGTGGTTGGTGGAGTCCTGGCTTGCTATAGCTTGCTAGTAACAGTG GCCTTTATTATTTTCTGGGTGAGGAGTAAGAGGAGCAGGCTCCTGCACAGT GACTACATGAACATGACTCCCCGCCGCCCCGGGCCCACCCGCAAGCATTAC CAGCCCTATGCCCCACCACGCGACTTCGCAGCCTATCGCTCCCTGAGAGTG AAGTTCAGCAGGAGCGCAGACGCCCCCGCGTACCAGCAGGGCCAGAACCAG CTCTATAACGAGCTCAATCTAGGACGAAGAGAGGAGTACGATGTTTTGGAC AAGAGACGTGGCCGGGACCCTGAGATGGGGGGAAAGCCGAGAAGGAAGAAC CCTCAGGAAGGCCTGTACAATGAACTGCAGAAAGATAAGATGGCGGAGGCC TACAGTGAGATTGGGATGAAAGGCGAGCGCCGGAGGGGCAAGGGGCACGAT GGCCTTTACCAGGGTCTCAGTACAGCCACCAAGGACACCTACGACGCCCTT CACATGCAGGCCCTGCCCCCTCGCTGA | Non-humanized anti-CCR7 CAR construct (Nucleic acid sequence of SEQ ID 34) |
| 43 | GCCGTGCAGCTTGTTGAGTCTGGTGGAGGATTGGTGCAGCCTAAGGAGTCA TTGAAAATCTCATGTGCAGCCTCTGGATTCACCTTCAGTAATGCTGCCATG TACTGGGTCCGCCAGGCTCCAGGAAAGGGTCTGGAATGGGTTGCTCGCATA AGAACTAAACCTAATAATTTTGCAACATATTATGCTGACTCAGTGAAAGGC AGATTCACCATCTCCAGAGATGATTCAAAAAGCATGGTCTACCTACAAATG GATAAGTTGAAAACTGAGGACACAGCCATGTATTATTGTACAGCTGGGAAG GATTACTTTGCTTACTGGGGCCAAGGCACTCTGGTCACTGTCTCTTCAG | Non-humanized VH (3D12) |
| 44 | GCCGTGCAGCTGGTGGAATCTGGCGGAGGACTGGTGCAGCCTAAAGAGAGC CTGAAGATCAGCTGTGCCGCCAGCGGCTTCACCTTTAGCAATGCCGCCATG TACTGGGTCCGACAGGCCCCTGGAAAAGGCCTGGAATGGGTTGCCCGGATC AGGACCAAGCCTAACAACTTCGCCACCTACTACGCCGACAGCGTGAAGGGC AGATTCACCATCAGCCGGGACGACAGCAAGAGCATGGTGTACCTGCAGATG GACAAGCTGAAAACCGAGGACACCGCTATGTACTACTGCACCGCCGGCAAG GACTACTTCGCCTATTGGGGACAGGGCACCCTGGTCACAGTGTCT | Non-humanized VH (3D12)_CO |
| 45 | GATATTGTGATGACCCAGGGTGCACTCCCCAATCCTGTCCCTTCTGGAGAG TCAGCTTCCATCACCTGCCAGTCTAGCAAGAGTCTGCTGTTCAGCGATGGC AAGACATACTTGAATTGGTATCTGCAGAGGCCAGGACAGTCTCCTCAGTTC CTGATCTATTGGATGTCTACCCGTGCATCAGGAGTCTCAGACAGGTTCAGT GGCAGTGGGTCAGGAACAGATTTCACACTGAAAATCAGTAGAGTGGAGGCT GAGGATGTGGGTGTGTATTACTGTCAGCAATTTCTAGAGTTTCCATTCACG TTCGGCTCAGGGACGAAGTTGGAAATAAAAC | Non-humanized VL (3D12) |
| 46 | GATATCGTGATGACACAGGGCGCTCTGCCCAATCCTGTGCCTTCTGGCGAG AGCGCCTCTATCACCTGTCAGAGCAGCAAGTCCCTGCTGTTCAGCGACGGC AAGACCTACCTGAACTGGTATCTGCAGAGGCCCGGACAGAGCCCTCAGTTC CTGATCTACTGGATGAGCACCAGAGCCAGCGGCGTGTCCGATAGATTTTCT GGCAGCGGCTCCGGCACCGACTTCACCCTGAAGATTAGCAGAGTGGAAGCC GAGGACGTGGGCGTGTACTATTGCCAGCAGTTCCTGGAGTTCCCATTCACC TTCGGCAGCGGCACCAAGCTGGAAATCAA | Non-humanized VL (3D12)_CO |

A further aspect of the invention relates to a vector comprising a nucleic acid molecule as described herein, preferably a viral vector, such as a retroviral vector, lentiviral vector, or transposon, encoding a chimeric antigen receptor (CAR) polypeptide.

A further aspect of the invention relates to a cell, preferably a genetically modified immune cell, comprising a nucleic acid molecule or vector as described herein and/or expressing a CAR as described herein.

In embodiments, the genetically modified immune cell is a natural killer (NK) cell.

In a preferred embodiment, the immune cells intended for administering in treatment of the diseases mentioned herein are genetically modified with a nucleic acid as described herein, encoding and expressing the anti-CCR7 CAR as described herein, using a viral vector, in particular, a retroviral vector. Retroviral vectors have demonstrated high transduction efficiency in expanded NK cells, facilitating the development of CAR-NK cells. Additionally, the CRISPR/Cas9 technique provides a targeted and precise approach for gene integration, offering the potential to enhance NK cell anti-tumor functions by modifying specific genes involved in NK cell regulation.

In further embodiments of the invention, CRISPR/Cas and TALEN-mediated insertion of the CCR7 CAR encoding nucleic acid may be employed. CRISPR/Cas, known to a skilled person, which is adapted from a naturally occurring process in bacteria, may be employed to precisely and efficiently edit DNA to insert the appropriate coding sequences into the immune cell, preferably the NK cell of interest. Cas9, a protein that acts as a molecular pair of scissors, is guided to a specific DNA sequence by an associated RNA molecule (a guide RNA). When Cas9 arrives at its target location on the DNA, it facilitates a change in the local genetic code, affecting the function of that gene. CRISPR/Cas9 can deliver the CAR gene to a specific site within the NK cell genome, which may reduce the risk of gene insertion at incorrect or undesired locations.

In one embodiment, the immune cell is preferably selected from the group consisting of an NK cell or a T lymphocyte, more preferably an NK cell.

NK cells operate independently of HLA-matching, allowing them to function as allogeneic effectors without being sourced from a particular patient or a donor with a specific HLA match. NK cell-mediated cytotoxicity is regulated by a repertoire of activating and inhibitory receptors (Guillerey et al., 2016; Morvan and Lanier, 2016). Activating receptors include but are not limited to natural cytotoxic receptors (NCRs), NKG2D, CD16 (FcγRIIIa), FasL, TRAIL, and costimulatory receptors such as LFA-1, CD244 (2B4), and CD137 (41BB). These activating cell surface receptors have the capacity to trigger cytolytic programs, as well as cytokine and chemokine secretion via intracytoplasmic ITAMs such as in 2B4, 41BB, and/or via other transmembrane signaling adaptors (Li Y et al., Cell Stem Cell, 2018).

An additional and surprising aspect of the invention is the improved stability of the CAR, as disclosed herein. The CAR polypeptide can readily be stored for extended periods under appropriate conditions without losing binding affinity.

A further aspect of the invention relates to a genetically modified immune cell as described herein for use in the treatment of a medical disorder associated with the presence of pathogenic cells expressing CCR7, preferably semi-mature or mature dendritic cells, monocytes/macrophages, naive B cells and T cells, including T regulatory and central memory T cells.

In one embodiment, the medical disorder to be treated is T cell lymphoma, preferably T cell non-Hodgkin's lymphoma (T-NHL). In preferred embodiments, the medical disorder is a T cell non-Hodgkin's lymphoma, with or without a leukemic tumor cell dissemination.

In other embodiments, the medical disorder to be treated is a B cell derived lymphoproliferative disorder, selected preferably from the group consisting of chronic lymphatic leukemia (CLL), mantle cell lymphoma (MCL) and classical Hodgkin lymphoma (cHL).

In other embodiments, the medical disorder to be treated is a T cell-derived lymphoproliferative disorder, selected from the group consisting of T-cell prolymphocytic leukemia (T-PLL) and T cell leukemia central nervous system (CNS) infiltration.

In embodiments, the disease to be treated is characterized by the presence of pathogenic cells expressing CCR7. For example, CCR7 is highly expressed in a variety of B cell malignancies, including chronic lymphocytic leukemia (CLL), mantle cell lymphoma (MCL), and classical Hodgkin lymphoma (cHD). Strong CCR7 expression has also been detected in T-cell prolymphocytic leukemia (T-PLL). Malignancies of skin-homing T cells, the cutaneous T-cell lymphomas (CTCLs), represent a very heterogeneous group of non-Hodgkin lymphomas encompassing leukemic variants such as Sezary syndrome (SS) and Mycosis fungoides (MF) that also highly express CCR7. In classical Hodgkin Lymphoma (cHL), CCR7 is expressed in tumor cell lines and primary tissues, with moderate to high functional expression. While previous evidence did not indicate CCR7 mRNA overexpression in T-PLL samples, it was recently found that CCR7 surface levels are significantly elevated in a high proportion (86.5%) of T-PLL cases.

A further aspect of the invention relates to a cell, preferably a genetically modified immune cell as described herein, expressing the inventive CAR for use as a medicament in the treatment of an autoantibody-dependent autoimmune disease.

Only recently, CAR-NK cells have also been discussed as a targeted approach to treat autoantibody-mediated diseases (Müzes G, Cells, 2023). The ability to target CCR7 would inhibit co-localization of autoreactive B cells and T cells, which would be of great benefit for the treatment of numerous autoimmune diseases.

The invention relates further to methods of treatment of the medical conditions described herein, comprising typically the administration of a therapeutically effective amount of the CAR, or an immune cell-expressing said CAR, to a patient in need of said treatment.

The features and embodiments of the invention relating to the CAR or components of the CAR also relate to any and all other aspects of the invention, such as the cells, nucleic acid molecules, methods of treatment, and so forth. Any feature or embodiment used to define one aspect of the invention may be used to define, and are considered disclosed in context to, any other aspect of the invention, as would be understood by a person skilled in the art.

### DETAILED DESCRIPTION OF THE INVENTION

### Chimeric Antigen Receptors:

According to the present invention, a chimeric antigen receptor polypeptide (CAR) comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules derived from NK cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from CD28. The term "generation" refers to the number of intracellular activating signals. First-generation CAR-NK cells only contain one activating signal, namely the CD3ζ signal. The second and third-generation CAR-NK bear one and two additional co-stimulatory signals, respectively. The co-stimulatory molecules are usually derived from the CD28 family (including CD28 and ICOS), the tumor necrosis factor receptor (TNFR) family of genes (including 4-1 BB, OX40, and CD27), or signaling lymphocytic activation molecule (SLAM)-related receptor family (comprising 2B4).

In various embodiments, genetically engineered receptors that redirect the cytotoxicity of immune effector cells toward T cells are provided. These genetically engineered receptors are referred to herein as chimeric antigen receptors (CARs). CARs are molecules that combine antibody-based specificity for a desired antigen (e.g., CCR7) with a NK cell receptor-activating intracellular domain to generate a chimeric protein that exhibits a specific anti-CCR7 cellular immune activity. As used herein, the term "chimeric" describes being composed of parts of different proteins or DNAs from different origins.

CARs contemplated herein comprise an extracellular domain (also referred to as a binding domain or antigen-binding domain) that binds to CCR7, a transmembrane domain, and an intracellular domain, or intracellular signaling domain. Engagement of the anti-CCR7 antigen-binding domain of the CAR with CCR7 on the surface of a target cell results in clustering of the CAR and delivers an activation stimulus to the CAR-containing cell. The main characteristic of CARs is their ability to redirect immune effector cell specificity, thereby triggering proliferation, cytokine production, phagocytosis, or production of molecules that can mediate cell death of the target antigen-expressing cell in a major histocompatibility complex (MHC) independent manner, exploiting the cell-specific targeting abilities of monoclonal antibodies, soluble ligands or cell-specific co-receptors.

In various embodiments, a CAR comprises an extracellular binding domain that comprises a humanized CCR7-specific binding domain, a transmembrane domain, and one or more intracellular signaling domains. In particular embodiments, a CAR comprises an extracellular binding domain comprising a humanized anti-CCR7 antigen-binding fragment, a spacer domain, a transmembrane domain, and one or more intracellular signaling domains.

The "extracellular antigen-binding domain" or "extracellular binding domain" are used interchangeably and provide a CAR with the ability to bind to the target antigen of interest, CCR7 specifically. The binding domain may be derived from a natural, synthetic, semi-synthetic, or recombinant source. Preferred are scFv domains.

"Specific binding" is to be understood as via one skilled in the art, whereby the skilled person is aware of various experimental procedures that can be used to test binding and binding specificity. Methods for determining equilibrium association or equilibrium dissociation constants are known in the art. Cross-reaction or background binding may be inevitable in many protein-protein interactions; this is not to detract from the "specificity" of the binding between CAR and epitope. "Specific binding" describes the binding of an anti-CCR7 antibody or antigen-binding fragment thereof (or a CAR comprising the same) to CCR7 at greater binding affinity than background binding. The term "directed against" is also applicable when considering the term "specificity" in understanding the interaction between antibodies and epitopes.

An "antigen (Ag)" refers to a compound, composition, or substance that can stimulate the production of antibodies or a T and B cell response in an animal. In particular embodiments, the target antigen is an epitope of a CCR7 polypeptide. An "epitope" refers to the region of an antigen to which a binding agent binds. Epitopes can be formed both from contiguous amino acids or noncontiguous amino acids juxtaposed by the tertiary folding of a protein.

"Single-chain Fv" or "scFv" antibody fragments comprise the VH and VL domains of an antibody, wherein these domains are present in a single polypeptide chain and either orientation (e.g., VL-VH or VH-VL). Generally, the scFv polypeptide further comprises a polypeptide linker between the VH and VL domains, which enables the scFv to form the desired structure for antigen binding. In preferred embodiments, a CAR contemplated herein comprises an antigen-specific binding domain that is a scFv and may be a murine, human, or humanized scFv. Single-chain antibodies may be cloned from the V region genes of a hybridoma specific for a desired target. In particular embodiments, the antigen-specific binding domain is a humanized scFv that binds a human CCR7 polypeptide. An illustrative example of a variable heavy chain that is suitable for constructing anti-CCR7 CARs contemplated herein includes but is not limited to the amino acid sequences set forth in SEQ ID NO: 9 to 16. An illustrative example of a variable light chain that is suitable for constructing anti-CCR7 CARs contemplated herein includes but is not limited to the amino acid sequences set forth in SEQ ID NO: 17 to 27.

### Antibodies and antibody fragments:

The CAR comprises an extracellular antigen-binding domain, comprising an antibody or antibody fragment that binds the CCR7 polypeptide. Antibodies or antibody fragments of the invention, therefore, include but are not limited to polyclonal, monoclonal, bispecific, human, humanized, or chimeric antibodies, single chain fragments (scFv), single variable fragments (ssFv), single domain antibodies (such as VHH fragments from nanobodies), Fab fragments, F(ab)2 fragments, fragments produced by a Fab expression library, anti-idiotypic antibodies and epitope-binding fragments or combinations thereof of any of the above, provided that they retain similar binding properties of the CAR described herein, preferably comprising the corresponding CDRs, or VH and VL regions as described herein. Also mini-antibodies and multivalent antibodies such as diabodies, triabodies, tetravalent antibodies and peptabodies can be used in a method of the invention. The immunoglobulin molecules of the invention can be of any class (i.e., IgG, IgE, IgM, IgD, and IgA) or subclass of immunoglobulin molecules. Thus, the term antibody, as used herein, also includes antibodies and antibody fragments comprised by the CAR of the invention, either produced by modifying whole antibodies or synthesized de novo using recombinant DNA methodologies.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, defining the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 110 or more amino acids, primarily responsible for antigen recognition. The terms "variable light chain" and "variable heavy chain" refer to these variable regions of the light and heavy chains, respectively. Optionally, the antibody or the immunological portion of the antibody can be chemically conjugated to or expressed as a fusion protein with other proteins.

The CARs of the invention are intended to bind against mammalian, in particular human, protein targets. Protein names may correspond to either mouse or human versions of a protein.

Affinities of binding domain polypeptides and CAR proteins according to the present disclosure can be readily determined using conventional techniques, e.g., by competitive ELISA (enzyme-linked immunosorbent assay), or by binding association, or displacement assays using labeled ligands, or using a surface-plasmon resonance device such as Biacore.

Humanized antibodies comprising one or more CDRs of the invention or one or more CDRs derived from said antibodies can be made using any methods known in the art. For example, four general steps may be used to humanize a monoclonal antibody. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. See, for example, U.S. Pat. Nos. 4,816,567; 5,807,715; 5,866,692; 6,331,415; 5,530,101; 5,693,761; 5,693,762; 5,585,089; 6,180,370; 5,225,539; 6,548,640.

The term humanized antibody means that at least a portion of the framework regions, and optionally a portion of CDR regions or other regions involved in binding, of immunoglobulin is derived from or adjusted to human immunoglobulin sequences. The humanized, chimeric, or partially humanized versions of the mouse monoclonal antibodies can, for example, be made employing recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively, the monoclonal antibodies used in the method of the invention may be human monoclonal antibodies. Human antibodies can be obtained, for example, using phage-display methods (WO 91/17271; WO 92/01047).

As used herein, humanized antibodies also refer to forms of non-human (e.g., murine, rat, goat, sheep, camel, llama, shark) antibodies that are specific chimeric immunoglobulins, immunoglobulin chains, or fragments thereof (such as Fv, Fab, Fab', F(ab')2 or other antigen-binding subsequences of antibodies) that contain minimal sequence derived from non-human immunoglobulin.

As used herein, human, humanized antibody, or antibody fragment means an antibody having an amino acid sequence corresponding to that of an antibody produced by a human and/or has been made using any of the techniques for making human antibodies known in the art or disclosed herein. Human antibodies or fragments thereof can be selected by competitive binding experiments or otherwise to have the same epitope specificity as a particular mouse antibody. Surprisingly, the humanized antibodies of the present invention share the beneficial functional properties of the mouse antibodies to a large extent. Human polyclonal antibodies can also be provided as serum from humans immunized with an immunogenic agent. Optionally, such polyclonal antibodies can be concentrated by affinity purification using amyloid fibrillar and/or non-fibrillar polypeptides or fragments thereof as an affinity reagent. Monoclonal antibodies can be obtained from serum according to the technique described in WO 99/60846.

### Variable Regions and CDRs

A variable region of an antibody refers to the variable region of the antibody light chain or the variable region of the antibody heavy chain, either alone or in combination. The variable regions of the heavy and light chains each consist of four framework regions (FR) connected by three complementarity determining regions (CDRs), also known as hypervariable regions. The CDRs in each chain are held together near the FRs and, with the CDRs from the other chain, contribute to the formation of the antigen-binding site of antibodies.

There are a number of techniques available for determining CDRs, such as an approach based on cross-species sequence variability (i.e., Kabat et al. Sequences of Proteins of Immunological Interest, (5th ed., 1991, National Institutes of Health, Bethesda Md.)); and an approach based on crystallographic studies of antigen-antibody complexes (Al-Lazikani et al. (1997) J. Molec. Biol. 273:927-948). Alternative approaches include the IMGT international ImMunoGeneTics information system (Marie-Paule Lefranc). The Kabat definition is based on sequence variability and is the most commonly used method. The Chothia definition is based on the location of the structural loop regions, whereas the AbM definition is a compromise between the two used by Oxford Molecular's AbM antibody modeling software (refer to www.bioinf.org.uk: Dr. Andrew C.R. Martin's Group). As used herein, a CDR may refer to CDRs defined by one or more approaches or by a combination of these approaches.

In some embodiments, the invention provides an antibody or fragment thereof incorporated into a CAR, wherein said antibody or fragment thereof comprises at least one CDR, at least two, at least three, or more CDRs that are substantially identical to at least one CDR, at least two, at least three, or more CDRs of the antibody of the invention. Other embodiments include antibodies that have at least two, three, four, five, or six CDR(s) that are substantially identical to at least two, three, four, five, or six CDRs of the antibodies of the invention or derived from the antibodies of the invention. In some embodiments, at least one, two, three, four, five, or six CDR(s) are at least about 70%, 75%, 85%, 86%, 87%, 88%, 89%, 90%, 95%, 96%, 97%, 98%, or 99% identical to at least one, two or three CDRs of the antibody of the invention. It is understood that, for purposes of this invention, binding specificity and/or overall activity is generally retained. However, the extent of activity may vary compared to said antibody (may be greater or lesser).

### Additional components of the CAR

In certain embodiments, the CARs contemplated herein may comprise linker residues between the various domains, added for appropriate spacing and conformation of the molecule, for example, a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. CARs contemplated herein may comprise one, two, three, four, or five or more linkers. In particular embodiments, the length of a linker is about 1 to about 25 amino acids, about 5 to about 20 amino acids, or about 10 to about 20 amino acids, or any intervening length of amino acids.

Illustrative examples of linkers include glycine polymers, glycine-serine polymers, glycine-alanine polymers, alanine-serine polymers, and other flexible linkers known in the art, such as the Whitlow linker. Glycine and glycine-serine polymers are relatively unstructured and may serve as a neutral tether between domains of fusion proteins such as the CARs described herein.

In particular embodiments, the binding domain of the CAR is followed by one or more "spacers" or "spacer polypeptides," which refers to the region that moves the antigen binding domain away from the effector cell surface to enable proper cell/cell contact, antigen binding, and activation. In certain embodiments, a spacer domain is a portion of an immunoglobulin, including, but not limited to, one or more heavy chain constant regions, e.g., CD8a, CH2, and CH3. The spacer domain can include the amino acid sequence of a naturally occurring immunoglobulin hinge region or an altered immunoglobulin hinge region. In one embodiment, the spacer domain comprises the CH2 and CH3 domains of IgG1. In one embodiment, the Fc-binding domain of such a spacer/hinge region is mutated to prevent binding of the CAR to Fc-receptors expressed on macrophages and other innate immune cells.

In some embodiments, the CAR's binding domain may be followed by one or more "hinge domains," which play a role in positioning the antigen binding domain away from the effector cell surface to enable proper cell/cell contact, antigen binding, and activation. A CAR may comprise one or more hinge domains between the binding and transmembrane domains (TM). The hinge domain may be derived either from a natural, synthetic, semi-synthetic, or recombinant source. The hinge domain can include the amino acid sequence of a naturally occurring immunoglobulin hinge region or an altered immunoglobulin hinge region. Illustrative hinge domains suitable for use in the CARs described herein include but are not limited to CD8 alpha, CD8, Fc, IgG4, IgG1, DAP12, and CD28, which may be wild-type hinge regions from these molecules or may be altered. In another embodiment, the hinge domain comprises an IgG1-delta domain.

The "transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. The TM domain may be a type I transmembrane protein and may include CD3ζ, CD28, CD8, CD16, NKp44, NKp46, NKG2D, DNAM-1 and 2B4. In one embodiment, the CARs contemplated herein comprise a TM domain derived from CD28.

In particular embodiments, CARs contemplated herein comprise an intracellular signaling domain. An "intracellular signaling domain" refers to the part of a CAR that participates in transducing the message of effective anti-CCR7 CAR binding to a human CCR7 polypeptide into the interior of the immune effector cell to elicit effector cell function, e.g., activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of an immune effector cell. The effector function of the NK cell, for example, may be cytolytic activity or help or activity, including the secretion of a cytokine. Thus, the term "intracellular signaling domain" refers to the portion of a protein that transduces the effector function signal and directs the cell to perform a specialized function. CARs contemplated herein comprise one or more co-stimulatory signaling domains to enhance the efficacy, expansion, and/or memory formation of NK cells expressing CAR receptors. As used herein, the term "co-stimulatory signaling domain" refers to an intracellular signaling domain of a co-stimulatory molecule. Co-stimulatory molecules are cell surface molecules other than antigen receptors or Fc receptors that provide a second signal required for efficient activation and function of NK cells upon binding to antigen.

In one embodiment, the CAR comprises an intracellular domain comprising a co-stimulatory domain and a signaling (activation) domain. The CAR construct may, therefore, include an intracellular signaling domain (CD28, CD3zeta) of the native NK cell receptor complex and one or more co-stimulatory domains that provide a second signal to stimulate full NK cell activation. Co-stimulatory domains increase CAR-NK cell cytokine production and facilitate NK cell replication and persistence. Co-stimulatory domains have also been shown to potentially prevent CAR-NK cell exhaustion, increase NK cell antitumor activity, and enhance the survival of CAR-NK cells in patients. As a non-limiting example, CD28 is a member of the immunoglobulin (Ig) superfamily. It is constitutively expressed on NK cells and plays a critical role in NK cell activation by stimulating the PI3K-AKT signal transduction pathway. In one embodiment, the intracellular domain is a CD28 co-stimulatory domain. Other co-stimulatory domains comprising ICOS and OX40 can be combined with the CD3 zeta signaling (activation) domain.

### Polypeptides

"Peptide," "polypeptide," "polypeptide fragment," and "protein" are used interchangeably unless specified to the contrary and according to conventional meaning, i.e., as a sequence of amino acids. Polypeptides are not limited to a specific length, e.g., they may comprise a full-length protein sequence or a fragment of a full-length protein and may include post-translational modifications of the polypeptide, for example, glycosylations, acetylations, phosphorylations and the like, as well as other modifications known in the art, both naturally occurring and non-naturally occurring.

In various embodiments, the CAR polypeptides contemplated herein comprise a signal (or leader) sequence at the N-terminal end of the protein, which co-translationally or post-translationally directs the protein transfer. Polypeptides can be prepared using any of a variety of well-known recombinant and/or synthetic techniques. Polypeptides contemplated herein specifically encompass the CARs of the present disclosure or sequences that have deletions from, additions to, and/or substitutions of one or more amino acids of a CAR as disclosed herein.

An "isolated peptide" or an "isolated polypeptide" and the like, as used herein, refer to *in vitro* isolation and/or purification of a peptide or polypeptide molecule from a cellular environment and association with other components of the cell, i.e., it is not significantly associated with *in vivo* substances. Similarly, an "isolated cell" refers to a cell obtained from an *in vivo* tissue or organ and is substantially free of extracellular matrix.

### Nucleic acids

As used herein, the terms "polynucleotide" or "nucleic acid molecule" refers to a messenger RNA (mRNA), RNA, genomic RNA (gRNA), plus-strand RNA (RNA(+)), minus-strand RNA (RNA(-)), genomic DNA (gDNA), complementary DNA (cDNA) or recombinant DNA. Polynucleotides include single and double-stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence. In various illustrative embodiments, the present invention contemplates, in part, polynucleotides comprising expression vectors, viral vectors, transfer plasmids and compositions, and cells comprising the same.

Polynucleotides can be prepared, manipulated, and/or expressed using any of a variety of well-established techniques known and available in the art. In order to express a desired polypeptide, a nucleotide sequence encoding the polypeptide can be inserted into an appropriate vector. Examples of vectors are plasmids, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus {e.g., SV40). Examples of expression vectors are pClneo vectors (Promega) for expression in mammalian cells; pLenti4/V5-DEST^{™}, pLenti6/V5-DEST^{™}, and pLenti6.2/V5-GW/lacZ (Invitrogen) for lentivirus-mediated gene transfer and expression in mammalian cells. In particular embodiments, the coding sequences of the chimeric proteins disclosed herein can be ligated into such expression vectors for the expression of the chimeric protein in mammalian cells. The "control elements" or "regulatory sequences" present in an expression vector are those non-translated regions of the vector - the origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, suitable transcription and translation elements may be used, including ubiquitous promoters and inducible promoters.

### Vectors

In particular embodiments, a cell (e.g., an immune effector cell, such as a NK cell) is transduced with a retroviral vector, e.g., a lentiviral vector, encoding a CAR. For example, an immune effector cell is transduced with a vector encoding a CAR that comprises a humanized anti-CCR7 antibody or antigen-binding fragment that binds a CCR7 polypeptide with a transmembrane and intracellular signaling domain, such that these transduced cells can elicit a CAR-mediated cytotoxic response.

Retroviruses are a common tool for gene delivery. In particular embodiments, a retrovirus is used to deliver a polynucleotide encoding a chimeric antigen receptor (CAR) to a cell. As used herein, the term "retrovirus" refers to an RNA virus that reverse transcribes its genomic RNA into a linear double-stranded DNA copy and subsequently covalently integrates its genomic DNA into a host genome. Once the virus is integrated into the host genome, it is referred to as a "provirus." The provirus serves as a template for RNA polymerase II and directs the expression of RNA molecules, which encode the structural proteins and enzymes needed to produce new viral particles.

Illustrative retroviruses suitable for use in particular embodiments, include, but are not limited to: Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus.

As used herein, the term "lentivirus" refers to a group (or genus) of complex retroviruses. Illustrative lentiviruses include but are not limited to HIV (human immunodeficiency virus, including HIV type 1 and HIV type 2), visna-maedi virus (VMV) virus, the caprine arthritis-encephalitis virus (CAEV), equine infectious anemia virus (EIAV), feline immunodeficiency virus (FIV), bovine immune deficiency virus (BIV), and simian immunodeficiency virus (SIV).

The term "vector" is used herein to refer to a nucleic acid molecule capable of transferring or transporting another nucleic acid molecule. The transferred nucleic acid is generally linked to, e.g., inserted into, the vector nucleic acid molecule. A vector may include sequences that direct autonomous replication in a cell or may include sequences sufficient to allow integration into host cell DNA. Useful vectors include, for example, plasmids (e.g., DNA plasmids or RNA plasmids), transposons, cosmids, bacterial artificial chromosomes, and viral vectors. Useful viral vectors include e.g., replication defective retroviruses and lentiviruses. In further embodiments of the invention, CRISPR/Cas and TALEN-mediated insertion of the CCR7 CAR encoding nucleic acid may be employed. Appropriate vectors for CRISPR/Cas and TALEN-mediated insertion are known to a skilled person.

As will be evident to one of skill in the art, the term "viral vector" is widely used to refer either to a nucleic acid molecule (e.g., a transfer plasmid) that includes virus-derived nucleic acid elements that typically facilitate the transfer of the nucleic acid molecule or integration into the genome of a cell or to a viral particle that mediates nucleic acid transfer. Viral particles typically include various viral components and sometimes host cell components in addition to nucleic acid(s).

The term viral vector may refer either to a virus or viral particle capable of transferring a nucleic acid into a cell or to the transferred nucleic acid itself. Viral vectors and transfer plasmids contain structural and/or functional genetic elements primarily derived from viruses. The term "retroviral vector" refers to a viral vector or plasmid containing structural and functional genetic elements, or portions thereof, that are primarily derived from a retrovirus.

As will be evident to one of skill in the art, the term "viral vector" is widely used to refer either to a nucleic acid molecule (e.g., a transfer plasmid) that includes virus-derived nucleic acid elements that typically facilitate the transfer of the nucleic acid molecule or integration into the genome of a cell or to a viral particle that mediates nucleic acid transfer. Viral particles typically include various viral components and sometimes host cell components in addition to nucleic acid(s).

The term viral vector may refer either to a virus or viral particle capable of transferring a nucleic acid into a cell or to the transferred nucleic acid itself. Viral vectors and transfer plasmids contain structural and/or functional genetic elements that are primarily derived from a virus. The term "retroviral vector" refers to a viral vector or plasmid containing structural and functional genetic elements, or portions thereof, that are primarily derived from a retrovirus.

In a preferred embodiment, the invention relates to a method for transfecting cells with an expression vector encoding a CAR. For example, in some embodiments, the vector comprises additional sequences, such as sequences that facilitate CAR expression, such as a promoter, enhancer, poly-A signal, and/or one or more introns. In preferred embodiments, the CAR-coding sequence is flanked by transposon sequences, such that a transposase allows the coding sequence to integrate into the genome of the transfected cell.

### Sequence Variants:

Sequence variants of the claimed nucleic acids, proteins, antibodies, antibody fragments, and/or CARs, for example, those defined by % sequence identity, that maintain similar binding properties of the invention are also included in the scope of the invention. Such variants, which show alternative sequences, maintain essentially the same binding properties, such as target specificity, as the specific sequences provided are known as functional analogs or functionally analogous. Sequence identity relates to the percentage of identical nucleotides or amino acids when carrying out a sequence alignment.

The recitation "sequence identity," as used herein, refers to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, He, Phe, Tyr, Trp, Lys, Arg, His, Asp, Glu, Asn, Gin, Cys, and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions, and other changes in sequence that fall under the described sequence identity are also encompassed in the invention.

Protein sequence modifications, which may occur through substitutions, are also included within the scope of the invention. Substitutions, as defined herein, are modifications made to the amino acid sequence of the protein, whereby one or more amino acids are replaced with the same number of (different) amino acids, producing a protein that contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, substituting one amino acid for another of similar properties. Such "conserved" amino acids can be natural or synthetic, which, because of size, charge, polarity, and conformation, can be substituted without significantly affecting the structure and function of the protein. Frequently, many amino acids may be substituted by conservative amino acids without deleteriously affecting the protein's function.

In general, the non-polar amino acids Gly, Ala, Val, Ile, and Leu; the non-polar aromatic amino acids Phe, Trp, and Tyr; the neutral polar amino acids Ser, Thr, Cys, Gln, Asn, and Met; the positively charged amino acids Lys, Arg and His; the negatively charged amino acids Asp and Glu, represent groups of conservative amino acids. This list is incomplete. For example, it is well known that Ala, Gly, Ser, and sometimes Cys can substitute for each other even though they belong to different groups.

Substitution variants have at least one amino acid residue in the antibody molecule removed and a different residue inserted. The sites of greatest interest for substitutional mutagenesis include the hypervariable regions, but FR alterations are also contemplated. If such substitutions result in a change in biological activity, then more substantial changes, denominated "exemplary substitutions" in the table immediately below, or as further described below in reference to amino acid classes, may be introduced, and the products screened.

### Potential amino acid substitutions:

| **Original residue** | **Preferred conservative substitutions** | **Examples of exemplary substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids but include synthetic ones. Commonly used synthetic amino acids are omega amino acids of various chain lengths and cyclohexyl alanine, which are neutral non-polar analogs; citrulline and methionine sulfoxide, which are neutral non-polar analogs; phenyl glycine which is an aromatic neutral analog; cysteic acid which is a negatively charged analog and ornithine which is a positively charged amino acid analog. Like the naturally occurring amino acids, this list is not exhaustive but merely exemplary of the well-known substitutions in the art.

"Codon optimization" involves gene engineering strategies that employ synonymous codon changes to enhance protein production, commonly utilized in the production of protein pharmaceuticals and nucleic acid therapies, including gene therapy, mRNA therapy, and DNA/RNA vaccines. Codon optimization methods may include deep learning, utilizing the concept of "codon boxes" to recode DNA sequences (Hongguang F et al., Sci Rep, 2020).

### Genetically modified cells and Immune cells

The present invention contemplates, in particular embodiments, cells genetically modified to express the CARs contemplated herein. These cells are contemplated for use in treating the medical conditions disclosed herein, such as T cell-related conditions. As used herein, the term "genetically engineered" or "genetically modified" refers to adding extra genetic material in the form of DNA or RNA to the total genetic material in a cell. The terms "genetically modified cells," "modified cells," and "redirected cells" are used interchangeably. As used herein, the term "gene therapy" refers to the introduction - permanently or transiently - of extra genetic material in the form of DNA or RNA into the total genetic material in a cell that restores, corrects, or modifies the expression of a gene, or to express a therapeutic polypeptide, e.g., a CAR. In particular embodiments, the CARs contemplated herein are introduced and expressed in immune effector cells to redirect their specificity to a target antigen of interest, e.g., a CCR7 polypeptide.

An "immune cell" or "immune effector cell" is any cell of the immune system that has one or more effector functions (e.g., cytotoxic cell-killing activity, secretion of cytokines, induction of ADCC and/or CDC). An immune effector cell can also be differentiated from iPSCs (induced pluripotent stem cells).

Immune effector cells of the invention can be autologous/autogeneic ("self') or non-autologous ("non-self," e.g., allogeneic, syngeneic, or xenogeneic). "Autologous," as used herein, refers to cells from the same subject representing a preferred embodiment of the invention. "Allogeneic," as used herein, refers to cells of the same species that differ genetically from the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic.

Illustrative immune effector cells used with the CARs contemplated herein include NK cells.

"Natural Killer (NK) cells," also known as large granular lymphocytes (LGL), are a vital component of the innate immune system, comprising 5-20% of circulating lymphocytes in humans. Functionally analogous to cytotoxic T cells in adaptive immunity, NK cells respond rapidly to virus-infected cells and tumors, acting approximately three days after infection. Unlike most immune cells, NK cells do not require antibodies or major histocompatibility complex (MHC) for target recognition, allowing for a swift immune response. Identified by the presence of CD56 and absence of CD3, NK cells differentiate from CD127+ common innate lymphoid progenitors and mature in various tissues before entering circulation. CD56bright NK cells, predominant in bone marrow and lymphoid tissues, exhibit immunoregulatory roles, while CD56dim NK cells in peripheral blood excel in cell killing. There are various sources from which NK cells can be derived, e.g., peripheral blood mononuclear cells, cord blood, immortalized cell lines, hematopoietic stem and progenitor cells (HSPCs), and induced pluripotent stem cells (iPSCs). All sources can provide clinically meaningful cell doses, are amenable to CAR receptor engineering, and have transitioned into in-human studies. NK cells express distinct receptors, including inhibitory receptors recognizing self-MHC I and activating receptors (Raftery MJ, Ann Rev of Cancer Biol, 2023). Activating molecules may include the natural cytotoxicity receptors, NKG2D, some CD94/NKG2 complexes, and CD16 (FcγIII). Activating molecules recognize partner ligands on cells except for CD16, a low-affinity Fc receptor responsible for antibody-dependent cellular cytotoxicity (ADCC). Inhibitory molecules may include the killer cell immunoglobulin-like receptor (KIR) family of molecules, some CD94/NKG2 complexes, and leukocyte Ig-like receptors (LIRs). These receptors govern NK cell activity, adjusting to environmental cues. NK cells contribute to both innate and adaptive immunity, demonstrating antigen-specific immunological memory.

For example, when reintroduced back to patients after autologous cell transplantation, the NK cells modified with the CAR of the invention as described herein may recognize and kill tumor cells.

As would be understood by the skilled person, other cells may also be used as immune effector cells with the CARs as described herein. In particular, immune effector cells include T cells, NK cells, neutrophils, and macrophages. Immune effector cells also include progenitors of effector cells wherein such progenitor cells can be induced to differentiate into immune effector cells *in vivo* or *in vitro.* Progenitors can be iPSCs that become immune effector cells under defined culture conditions.

The present invention provides methods for making the immune effector cells that express the CAR contemplated herein. In one embodiment, the method comprises transfecting or transducing immune effector cells isolated from an individual such that the immune effector cells express one or more CARs as described herein. In certain embodiments, the immune effector cells are isolated from an individual and genetically modified without further manipulation *in vitro.* Such cells can then be directly re-administered into the individual. In further embodiments, the immune effector cells are first activated and stimulated to proliferate *in vitro* prior to being genetically modified to express a CAR. In this regard, the immune effector cells may be cultured before and/or after being genetically modified (i.e., transduced or transfected to express a CAR contemplated herein).

In particular embodiments, prior to *in vitro* manipulation or genetic modification of the immune effector cells described herein, the source of cells is obtained from a subject. In particular embodiments, the CAR-modified immune effector cells comprise NK cells. NK cells can be obtained from several sources, including, but not limited to, peripheral blood mononuclear cells, bone marrow, lymph node tissue, cord blood, thymus tissue, tissue from a site of infection, ascites, pleural effusion, spleen tissue, and tumors. In certain embodiments, NK cells can be obtained from a unit of blood collected from a subject using any number of techniques known to the skilled person, such as sedimentation, e.g., FICOLL^{™} separation, magnetic bead labeling, antibody-conjugated bead-based methods such as MACSTM separation (Miltenyi). In one embodiment, cells from the circulating blood of an individual are obtained by apheresis. The apheresis product typically contains lymphocytes, including NK cells, T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or another suitable solution lacking calcium, magnesium, and most, if not all, other divalent cations. As would be appreciated by those of ordinary skill in the art, a washing step may be accomplished by methods known to those in the art, such as using a semiautomated flow through a centrifuge, for example, the Cobe 2991 cell processor, the Baxter CytoMate, or the like. After washing, the cells may be resuspended in various biocompatible buffers or other saline solutions with or without buffer. In certain embodiments, the undesirable components of the apheresis sample may be removed in the cell directly resuspended culture media.

In certain embodiments, NK cells are isolated from peripheral blood mononuclear cells (PBMCs) by lysing the red blood cells and depleting the monocytes, for example, by centrifugation through a PERCOLL^{™} gradient. Positive or negative selection techniques can further isolate a specific subpopulation of NK cells. One method for use herein is cell sorting and/or selection via negative magnetic immunoadherence or flow cytometry that uses a cocktail of monoclonal antibodies directed to cell surface markers present on the cells negatively selected.

PBMC may be directly genetically modified to express CARs using methods contemplated herein. In certain embodiments, after isolation of PBMC, NK cells are further isolated.

In some embodiments, the immune cells of the present invention, for example, the NK cells described herein, can be obtained from inducible pluripotent stem cells (iPSCs) using methods known to a skilled person.

Direct *in vitro* differentiation of engineered NK cells from pluripotent stem cells, such as inducible pluripotent stem cells, provides an unlimited source of cells that can be genetically modified to express the CAR of the present invention. In some embodiments, a so-called master iPSC line can be maintained, representing a renewable source for consistently and repeatedly manufacturing homogeneous cell products. In some embodiments, the transformation of a master iPSC cell line with the CAR encoding nucleic acid is contemplated prior to expansion and differentiation to the desired immune cell, preferably NK cell. NK cells can, for example, be generated from iPSCs, such that iPSCs could be modified with the CAR encoding nucleic acids and subsequently expanded and differentiated to NK cells for administration to the patient. Differentiation to the appropriate immune cell, such as an NK cell, could also be conducted from the iPSCs before transformation with CAR encoding nucleic acids and expansion prior to administration. All possible combinations of iPSC expansion, genetic modification, and expansion to provide suitable numbers of cells for administration are contemplated in the invention.

The immune effector cells, such as NK cells, can be genetically modified following isolation using known methods, or the immune effector cells can be activated and expanded (or differentiated in the case of progenitors) *in vitro* prior to being genetically modified. In a particular embodiment, the immune effector cells, such as NK cells, are genetically modified with the chimeric antigen receptors contemplated herein (e.g., transduced with a viral vector comprising a nucleic acid encoding a CAR) and then are activated and expanded *in vitro.* In various embodiments, NK cells can be activated and expanded before or after genetic modification to express a CAR using methods known to a skilled person.

In a further embodiment, a mixture of, e.g., one, two, three, four, five, or more, different expression vectors can be used in genetically modifying a donor population of immune effector cells wherein each vector encodes a different chimeric antigen receptor protein as contemplated herein. The resulting modified immune effector cells form a mixed population of modified cells, with a proportion of the modified cells expressing more than one different CAR protein.

In one embodiment, the invention provides a method of storing genetically modified murine (rat), human, or humanized CAR protein expressing immune effector cells that target a CCR7 protein, comprising cryopreserving the immune effector cells such that the cells remain viable upon thawing. A fraction of the immune effector cells expressing the CAR proteins can be cryopreserved by methods known in the art to provide a permanent source of such cells for the future treatment of patients afflicted with T cell-related conditions. The cryopreserved transformed immune effector cells can be thawed, grown, and expanded for more such cells when needed.

The terms "T cell" or "T lymphocyte" are art-recognized and are intended to include thymocytes, immature T lymphocytes, mature T lymphocytes, resting T lymphocytes, cytokine-induced killer cells (CIK cells), or activated T lymphocytes. Cytokine-induced killer (CIK) cells are typically CD3- and CD56-positive, non-major histocompatibility complex (MHC)-restricted, natural killer (NK)-like T lymphocytes. A T cell can be a T helper (Th; CD4+ T cell) cell, for example, a T helper 1 (Th1) or a T helper 2 (Th2) cell. The T cell can be a cytotoxic T cell (CTL; CD8+ T cell), CD4+CD8+ T cell, CD4 CD8 T cell, or any other subset of T cells. Other illustrative populations of T cells suitable for particular embodiments include naive T cells, memory T cells, and stem cell-like memory cells TSCM).

### Compositions and Formulations

The compositions contemplated herein may comprise one or more polypeptides, polynucleotides, vectors comprising the same genetically modified immune effector cells, etc., as contemplated herein. Compositions include, but are not limited to, pharmaceutical compositions. A "pharmaceutical composition" refers to a composition formulated in pharmaceutically acceptable or physiologically acceptable solutions for administration to a cell or an animal, either alone or in combination with one or more other therapy modalities. It will also be understood that, if desired, the compositions of the invention may be administered in combination with other agents as well, such as cytokines, growth factors, hormones, small molecules, chemotherapeutics, pro-drugs, drugs, antibodies, or other various pharmaceutically-active agents. There is virtually no limit to other components that may also be included in the compositions, provided that the additional agents do not adversely affect the ability of the composition to deliver the intended therapy.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals. Exemplary pharmaceutically acceptable carriers include, but are not limited to, to sugars, such as lactose, glucose, and sucrose; starches, such as corn starch and potato starch; cellulose, and its derivatives, such as sodium carboxymethyl cellulose, ethyl cellulose, and cellulose acetate; tragacanth; malt; gelatin; talc; cocoa butter, waxes, animal and vegetable fats, paraffin, silicones, bentonites, silicic acid, zinc oxide; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances employed in pharmaceutical formulations.

In particular embodiments, compositions of the present invention comprise an amount of CAR-expressing immune effector cells contemplated herein. As used herein, the term "amount" refers to "an amount effective" or "an effective amount" of a genetically modified therapeutic cell, e.g., NK cell, to achieve a beneficial or desired prophylactic or therapeutic result, including clinical results.

A "prophylactically effective amount" refers to an amount of a genetically modified therapeutic cell effective to achieve the desired prophylactic result. Typically, but not necessarily, since a prophylactic dose is used in subjects prior to or at an earlier stage of disease, the prophylactically effective amount is less than the therapeutically effective amount. The term prophylactic does not necessarily refer to a complete prohibition or prevention of a particular medical disorder. The term "prophylactic" also refers to reducing the risk of a certain medical disorder occurring or worsening its symptoms.

A "therapeutically effective amount" of a genetically modified therapeutic cell may vary according to factors such as the disease state, age, sex, and weight of the individual, and the ability of the stem and progenitor cells to elicit a desired response in the individual. A therapeutically effective amount is also one in which the therapeutically beneficial effects outweigh any toxic or detrimental effects of the virus or transduced therapeutic cells. The term "therapeutically effective amount" includes an amount that is effective to "treat" a subject (e.g., a patient). When a therapeutic amount is indicated, the precise amount of the compositions of the present invention to be administered can be determined by a physician considering individual differences in age, weight, tumor size, extent of infection or metastasis, and patient condition (subject).

Generally, a pharmaceutical composition comprising the NK cells described herein may be administered at a dosage of 10² to 10¹⁰ cells/kg body weight, preferably 10⁵ to 10⁷ cells/kg body weight, including all integer values within those ranges. The number of cells will depend upon the ultimate use for which the composition is intended, as will the type of cells included therein. For uses provided herein, the cells are generally in a volume of a liter or less, 500 mLs or less, or even 250 mLs or 100 mLs or less. Hence, the density of the desired cells is typically greater than 10⁶ cells/ml and generally greater than 10⁷ cells/ml, generally 10⁸ cells/ml or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. In some aspects of the present invention, particularly since all the infused cells will be redirected to a particular target antigen, lower numbers of cells may be administered. CAR-expressing cell compositions may be administered multiple times at dosages within these ranges. The cells may be allogeneic, syngeneic, xenogeneic, or autologous to the patient undergoing therapy.

Generally, compositions comprising the cells activated and expanded, as described herein, may be utilized in treating and preventing diseases that arise in immunocompromised individuals. In particular, compositions comprising the CAR-modified NK cells contemplated herein are used to treat T cell malignancies. The CAR-modified T cells of the present invention may be administered either alone or as a pharmaceutical composition in combination with carriers, diluents, excipients, and/or with other components or cell populations. In particular embodiments, pharmaceutical compositions contemplated herein comprise an amount of genetically modified NK cells in combination with one or more pharmaceutically or physiologically acceptable carriers, diluents, or excipients.

Pharmaceutical compositions of the present invention comprising a CAR-expressing immune effector cell population, such as NK cells, may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal, or intramuscular administration.

The liquid pharmaceutical compositions, whether they be solutions, suspensions, or other like forms, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methylparaben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes, or multiple dose vials made of glass or plastic. An injectable pharmaceutical composition is preferably sterile.

In a particular embodiment, compositions contemplated herein comprise an effective amount of CAR-expressing immune effector cells, alone or in combination with one or more therapeutic agents. Thus, the CAR-expressing immune effector cell compositions may be administered alone or with other known cancer treatments, such as radiation therapy, chemotherapy, transplantation, immunotherapy, hormone therapy, photodynamic therapy, etc. The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

### Therapeutic Methods

The genetically modified immune effector cells contemplated herein provide improved methods of adoptive immunotherapy for use in the treatment of medical disorders associated with the presence of pathogenic cells expressing CCR7 that include but are not limited to immunoregulatory conditions and hematological malignancies.

As used herein, "medical disorders associated with the presence of pathogenic cells expressing CCR7" refer to medical conditions, such as cancer or autoimmune disease, in which the cells involved in the pathophysiology of the disease demonstrate expression of CCR7 and preferably presentation of CCR7 on the cell surface. The expression of CCR7 can be determined by various methods known to a skilled person, for example, by isolating cells from a patient and assessing these by PCR using primers directed CCR7 transcripts, immunostaining with anti-CCR7 antibodies, or by analysis by flow cytometry. Such pathogenic cells may typically be pathogenic mature B cells and/or memory B cells, and/or pathogenic T cells and/or T follicular helper cells.

In particular embodiments, compositions comprising CAR-modified NK cells contemplated herein are used in the treatment of hematologic malignancies, including but not limited to B and T cell malignancies such as, for example, non-Hodgkin's lymphoma (NHL), such as B cell NHL or T cell non-Hodgkin's lymphoma, with or without a leukemic tumor cell dissemination.

Non-Hodgkin lymphoma encompasses a large group of cancers of lymphocytes (white blood cells). Non-Hodgkin lymphomas can occur at any age and are often marked by lymph nodes that are larger than usual, fever, and weight loss. Non-Hodgkin lymphomas can also present on extranodal sites, such as the central nervous system and mucosal tissues, including the lung, intestine, colon, and gut. There are many different types of non-Hodgkin lymphoma. For example, non-Hodgkin's lymphoma can be divided into aggressive (fast-growing) and indolent (slow-growing) types.

Non-Hodgkin lymphomas can be derived from B cells and T-cells. As used herein, the term "non-Hodgkin lymphoma" includes both "B cell" and "T cell" non-Hodgkin lymphoma. B cell non-Hodgkin lymphomas (NHL) include Burkitt lymphoma, chronic lymphocytic leukemia/small lymphocytic lymphoma (CLL/SLL), diffuse large B cell lymphoma, follicular lymphoma, immunoblastic large cell lymphoma, precursor B-lymphoblastic lymphoma, and mantle cell lymphoma. Lymphomas that occur after bone marrow or stem cell transplantation are usually B cell non-Hodgkin lymphomas.

T-cell lymphomas account for approximately 15 percent of all NHLs in the United States. There are many forms of T-cell lymphomas, such as angioimmunoblastic T-cell lymphoma (AITL), a mature T-cell lymphoma of blood or lymph vessel immunoblasts. Further forms of T cell lymphomas relate to cutaneous T cell lymphoma and T cell lymphoma with leukemic dissemination.

Chronic lymphocytic leukemia (CLL) can also be treated with the present CAR and is an indolent (slow-growing) cancer that causes a slow increase in immature white blood cells (B lymphocytes). Cancer cells spread through the blood and bone marrow and can also affect the lymph nodes or other organs such as the liver and spleen. CLL eventually causes the bone marrow to fail. A different presentation of the disease is called small lymphocytic lymphoma and localizes predominantly to secondary lymphoid organs, e.g., lymph nodes and spleen.

In one embodiment of the invention, the CAR or immune cell expressing said CAR is intended to treat an autoimmune disease, preferably an auto-antibody-dependent autoimmune disease, preferably an autoimmune disease with an inflammatory component.

The autoimmune disease to be treated is preferably selected from Takayasu Arteritis, Giant-cell arteritis, familial Mediterranean fever, Kawasaki disease, Polyarteritis nodosa, cutanous Polyarteritis nodosa, Hepatitis-associated arteritis, Behcet's syndrome, Wegener's granulomatosis, ANCA-vasculitidies, Churg-Strauss syndrome, microscopic polyangiitis, Vasculitis of connective tissue diseases, Hennoch-Sch6nlein purpura, Cryoglobulinemic vasculitis, Cutaneous leukocytoclastic angiitis, Tropical aortitis, Sarcoidosis, Cogan's syndrome, Wiskott-Aldrich Syndrome, Lepromatous arteritis, Primary angiitis of the CNS, Thromboangiitis obliterans, Paraneoplastic ateritis, Urticaria, Dego's disease, Myelodysplastic syndrome, Eythema elevatum diutinum, Hyperimmunoglobulin D, Allergic Rhinitis, Asthma bronchiale, chronic obstructive pulmonary disease, periodontitis, Rheumatoid Arthritis, atherosclerosis, Amyloidosis, Morbus Chron, Colitis ulcerosa, Autoimmune Myositis, Diabetes mellitus, Guillain-Barre Syndrome, histiocytosis, Osteoarthritis, atopic dermatitis, periodontitis, chronic rhinosinusitis, Psoriasis, psoriatic arthritis, Microscopic colitis, Pulmonary fibrosis, glomerulonephritis, Whipple's disease, Still's disease, erythema nodosum, otitis, cryoglobulinemia, Sjogren's syndrome, Lupus erythematosus, preferably systemic lupus erythematosus (SLE), aplastic anemia, Osteomyelofibrosis, chronic inflammatory demyelinating polyneuropathy, Kimura's disease, systemic sclerosis, chronic periaortitis, chronic prostatitis, idiopathic pulmonary fibrosis, chronic granulomatous disease, Idiopathic achalasia, bleomycininduced lung inflammation, cytarabine-induced lung inflammation, Autoimmunthrombocytopenia, Autoimmunneutropenia, Autoimmunhemolytic anemia, Autoimmunlymphocytopenia, Chagas' disease, chronic autoimmune thyroiditis, autoimmune hepatitis, Hashimoto's Thyroiditis, atropic thyroiditis, Graves disease, Autoimmune polyglandular syndrome, Autoimmune Addison Syndrome, Pemphigus vulgaris, Pemphigus foliaceus, Dermatitis herpetiformis, Autoimmune alopecia, Vitiligo, Antiphospholipid syndrome, Myasthenia gravis, Stiff-man syndrome, Goodpasture's syndrome, Sympathetic ophthalmia, Folliculitis, Sharp syndrome and/or Evans syndrome, in particular hay fever, periodontitis, atherosclerosis, rheumatoid arthritis, most preferably SLE.

Systemic lupus erythematosus (SLE), also known as lupus, is an autoimmune disease in which the body's immune system attacks healthy tissue in various parts of the body. Symptoms vary between people and may be mild to severe. Common symptoms include painful and swollen joints, fever, chest pain, hair loss, mouth ulcers, swollen lymph nodes, feeling tired, and a red rash which is most commonly on the face.

As used herein, the terms "individual" and "subject" are often used interchangeably and refer to any animal that exhibits a symptom of a disease, disorder, or condition that can be treated with the gene therapy vectors, cell-based therapeutics, and methods disclosed elsewhere herein. In preferred embodiments, a subject includes any animal that exhibits symptoms of a disease, disorder, or condition of the hematopoietic system, e.g., a T cell malignancy, that can be treated with the cell-based therapeutics and methods disclosed herein. Suitable subjects include laboratory animals (such as mice, rats, rabbits, or guinea pigs), farm animals, and domestic animals or pets (such as cats or dogs). Non-human primates and, preferably, human patients are included. Typical subjects include human patients who have a B cell malignancy, have been diagnosed with a T cell malignancy, or are at risk of having a T cell malignancy.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms.

As used herein, "prevent" and similar words such as "prevented," "preventing," or "prophylactic," etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms, and/or burden of a disease or condition prior to the onset or recurrence of the disease or condition.

In one embodiment, a method of treating a T cell-related condition in a subject in need thereof comprises administering an effective amount, e.g., a therapeutically effective amount of a composition comprising genetically modified immune effector cells contemplated herein. The quantity and frequency of administration will be determined by such factors as the condition of the patient and the type and severity of the patient's disease, although clinical trials may determine appropriate dosages.

The compositions contemplated herein may be administered conveniently, including by aerosol inhalation, injection, ingestion, transfusion, implantation, or transplantation. In a preferred embodiment, compositions are administered parenterally. The phrases "parenteral administration" and "administered parenterally," as used herein, refer to modes of administration other than enteral and topical administration, usually by injection, and includes, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion. In one embodiment, the compositions contemplated herein are administered to a subject by direct injection into a tumor, lymph node, or site of infection.

### FIGURES

The invention is demonstrated by way of example in the following figures. The figures are to provide a further description of potentially preferred embodiments that enhance the support of one or more non-limiting embodiments of the invention.

### Brief description of the figures:

**Figure 1****:** Schematic representation of preferred CAR construct.
**Figure 2****:** List of preferred constructs and potential combinations of the various structural elements of the CARs as described herein.
**Figure 3****:** Sequences of complementary determining regions (CDRs) and framework regions (FRs) sequences of the rat (A) VH and (B) VL.
**Figure 4****:** Sequence depiction of the DNA sequence encoding the mAb binding regions rat VH.
**Figure 5****:** Sequence depiction of the DNA sequence encoding the mAb binding regions rat VL.
**Figure 6****:** Sequence depiction of the DNA sequence encoding the mAb binding regions rat VH, showing a sequence comparison between the original DNA sequence encoding the rat VH and the humanized VH sequence.
**Figure 7****:** Sequence depiction of the DNA sequence encoding the mAb binding regions rat VL, showing a sequence comparison between the original DNA sequence encoding the rat VL and the humanized VL sequence.
**Figure 8****:** VH-region mutations and amino acid (AA) changes.
**Figure 9****:** VL-region mutations and amino acid (AA) changes.
**Figure 10****:** GeneArt^{™} Plasmid with the rat CCR7 scFV sequence.
**Figure 11****:** Plasmid with the CCR7 CAR sequence.
**Figure 12****:** Cloning and generation of the anti-CCR7 CAR construct.
**Figure 13** CCR7 CAR expression on human primary NK cells following retroviral transduction.
**Figure 14****:** CCR7 surface expression on T-NHL cell lines.
**Figure 15****:** Co-cultures of CAR-transduced human NK cells with different target cell lines show specific NK cell activation by distinct CCR7+ T-NHL and control cell lines.
**Figure 16****:** Functional *in vitro* co-cultivation and CD107a analysis as a measure for degranulation.
**Figure 17****:** Cytotoxicity assays reveal selective killing of CCR7+ cell lines.
**Figure 18****:** Quantitative determination of CCR7 density per cell on primary blood leukocyte cell subpopulations was performed by flow cytometry and a CCR7-specific antibody.
**Figure 19****:** CCR7 surface expression by anti-CCR7 immunostaining and flow cytometry analysis.
**Figure 20****:** Co-cultures of CAR-transduced human NK cells with CCR7-negative primary cells of different human tissues and the CCR7-cell line REH as targets.
**Figure 21****:** Direct staining with the rat antibody 3D12 shows no positive signal for CCR7 expression on B cells but detects CCR7 positivity on T cells, whereas the mouse antibody G043H7 detects CCR7 on both types of lymphocytes.
**Figure 22****:** Development of a T-NHL xenograft model.

### Detailed description of the figures:

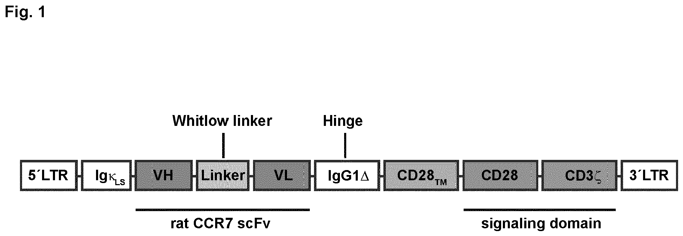

**Figure 1****:** Schematic representation of preferred CAR construct. The preferred construct of the invention comprises variants of the 3D12 single chain variable (scFv) heavy (VH) and light (VL) chains. LTR, long terminal repeat; TM, transmembrane region.

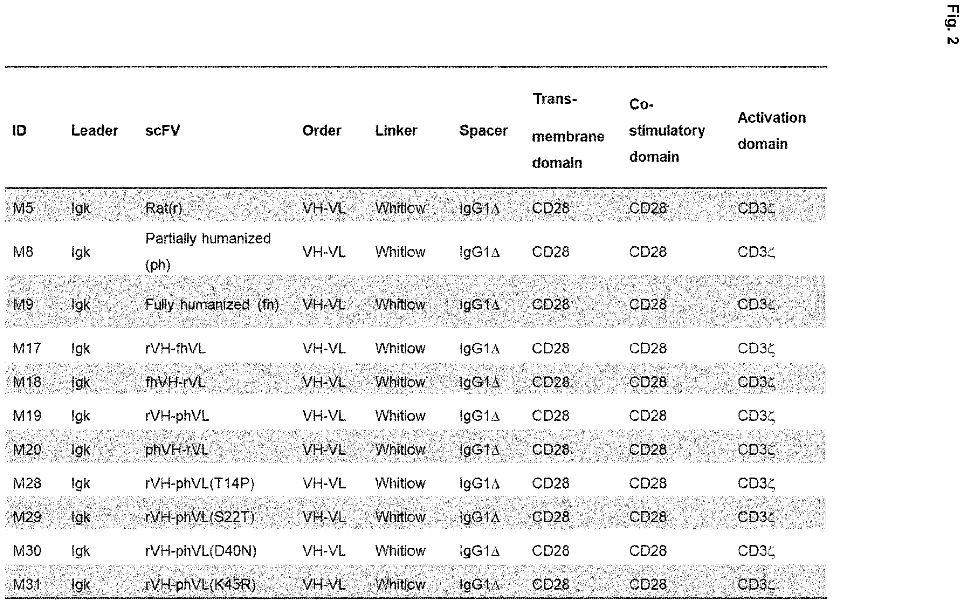

**Figure 2****:** List of preferred constructs and potential combinations of the various structural elements of the CARs as described herein; r, rat sequence; fh, fully humanized sequence; ph, partially humanized.

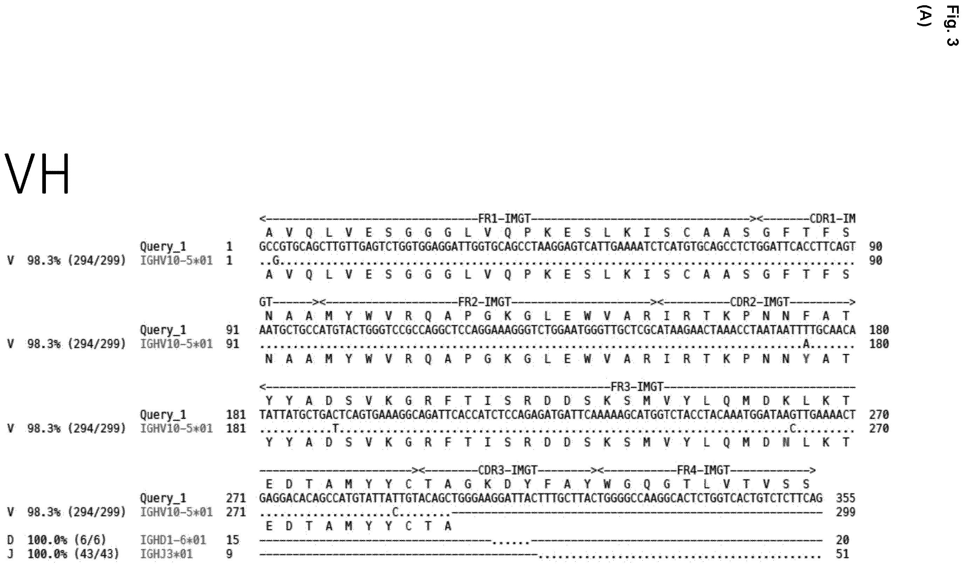

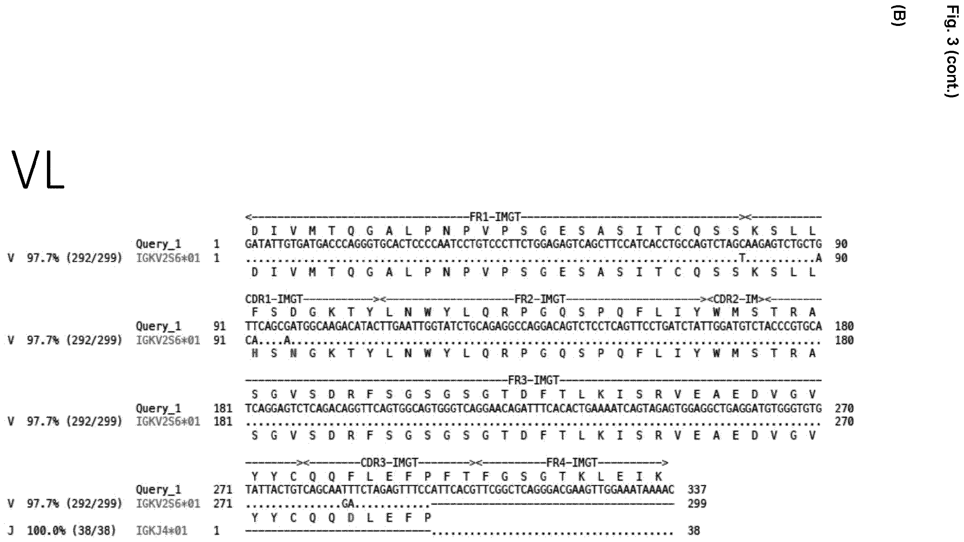

**Figure 3****:** Sequences of complementary determining regions (CDRs) and framework regions (FRs) sequences of the rat (A) VH and (B) VL. The Immunogenetics Database IMGT/V-QUEST was interrogated to annotate the CDR and FR sequences of the rat 3D12 VH (A) and VL (B) chains. Somatic hypermutations are indicated in bold.

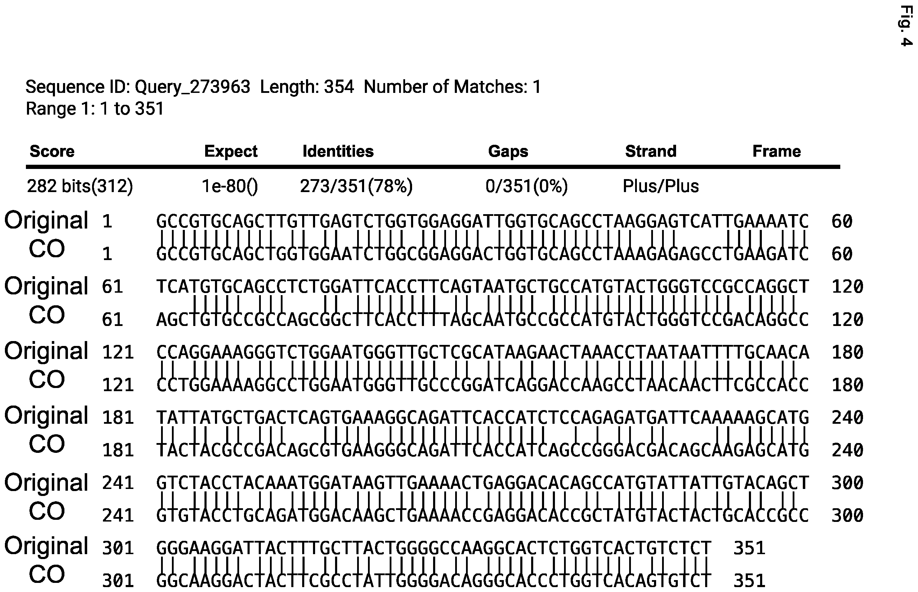

**Figure 4****:** Sequence depiction of the DNA sequence encoding the mAb binding regions rat VH. In particular, it shows a sequence comparison between the original and codon optimized (CO) DNA sequences encoding the rat VH.

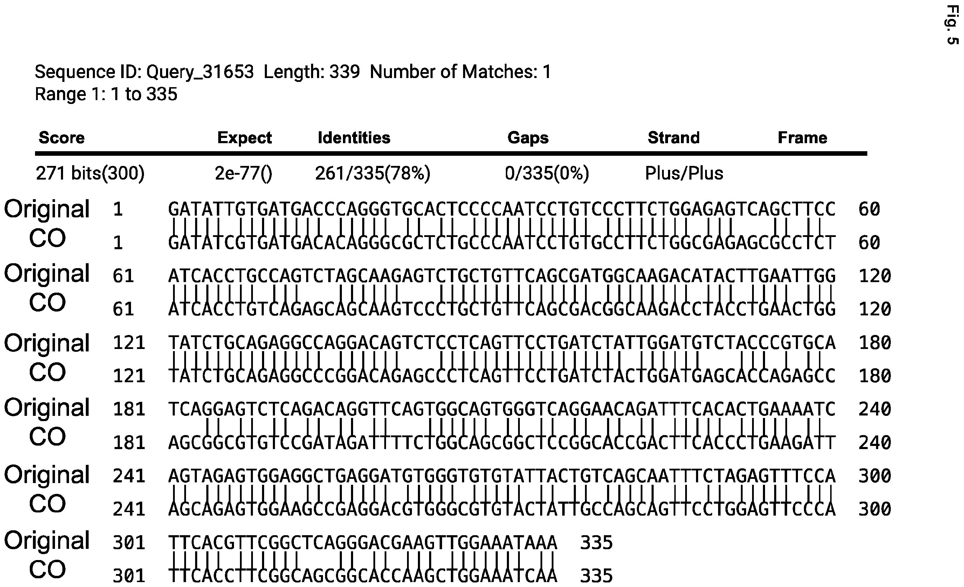

**Figure 5****:** Sequence depiction of the DNA sequence encoding the mAb binding regions rat VL. It shows a sequence comparison between the original and codon-optimized (CO) DNA sequences encoding the rat VL.

**Figure 6****:** Sequence depiction of the DNA sequence encoding the mAb binding regions rat VH, showing a sequence comparison between the original DNA sequence encoding the rat VH and the humanized VH sequence. The humanized VH sequence shares 78% homology with the rat VH sequence.

**Figure 7****:** Sequence depiction of the DNA sequence encoding the mAb binding regions rat VL, showing a sequence comparison between the original DNA sequence encoding the rat VL and the humanized VL sequence. The humanized VL sequence shares 78% homology with the rat VL sequence.

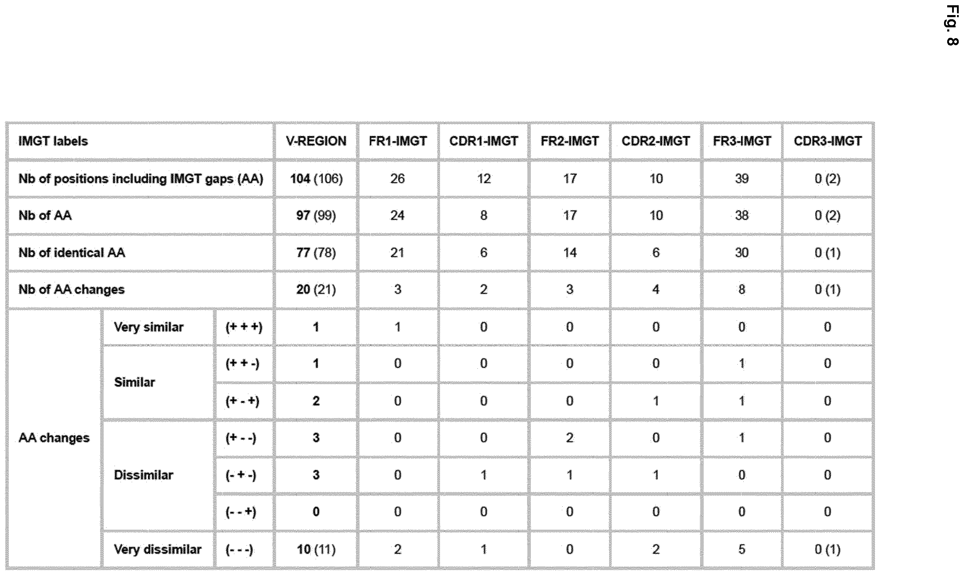

**Figure 8****:** VH-region mutations and amino acid (AA) changes. The aa changes are qualified as very similar: (+ + +), similar: (+ + -) or (+ - +), dissimilar: (+ - -) or (- + -) or (- - +), very dissimilar: (- - -).

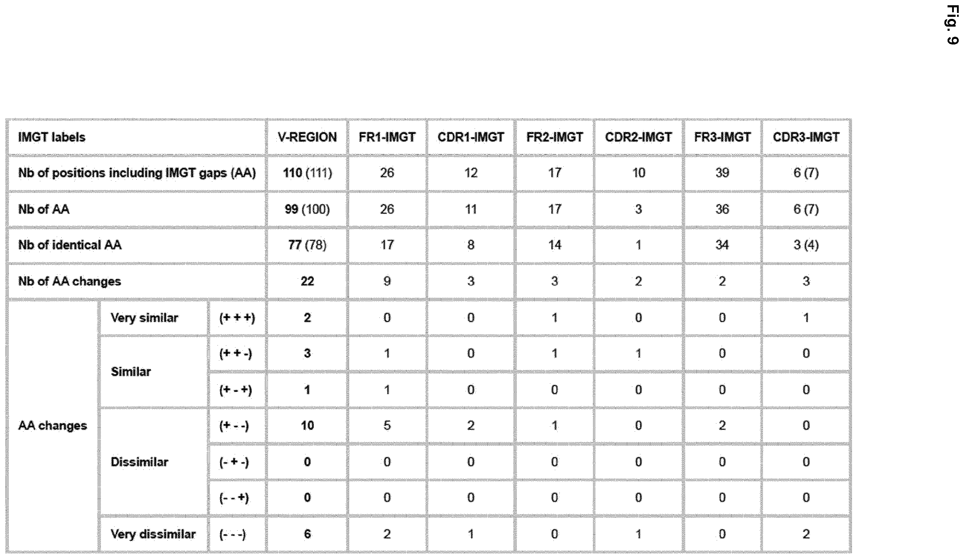

**Figure 9****:** VL-region mutations and amino acid (AA) changes. The aa changes are qualified as very similar: (+ + +), similar: (+ + -) or (+ - +), dissimilar: (+ - -) or (- + -) or (- - +), very dissimilar: (- - -).

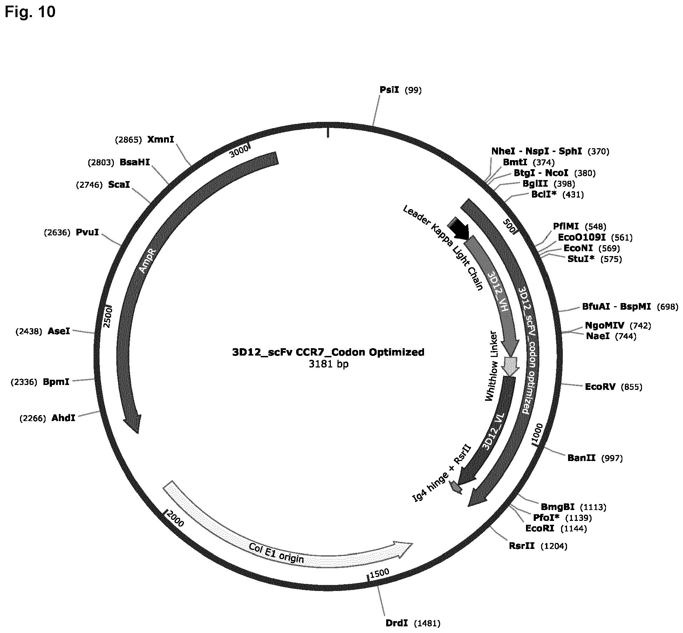

**Figure 10****:** GeneArt^{™} Plasmid with the rat CCR7 scFV sequence.

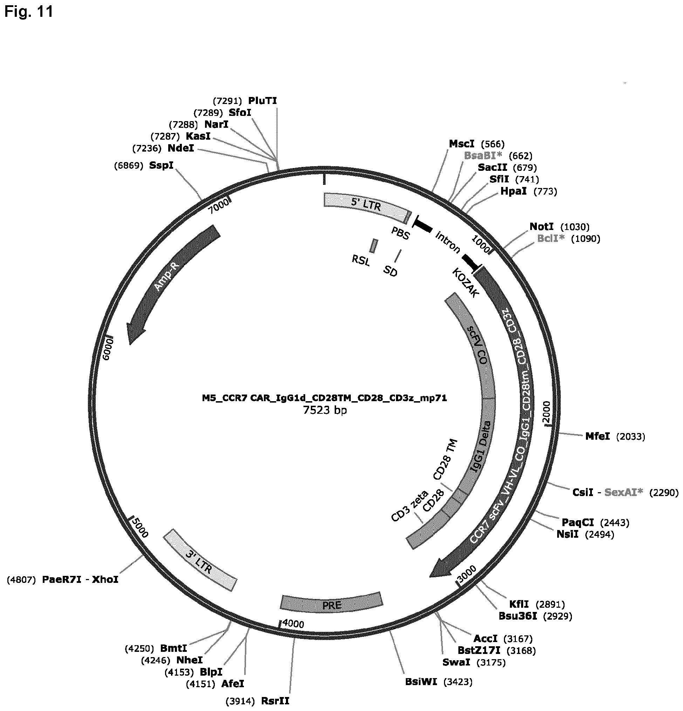

**Figure 11****:** Plasmid with the CCR7 CAR sequence.

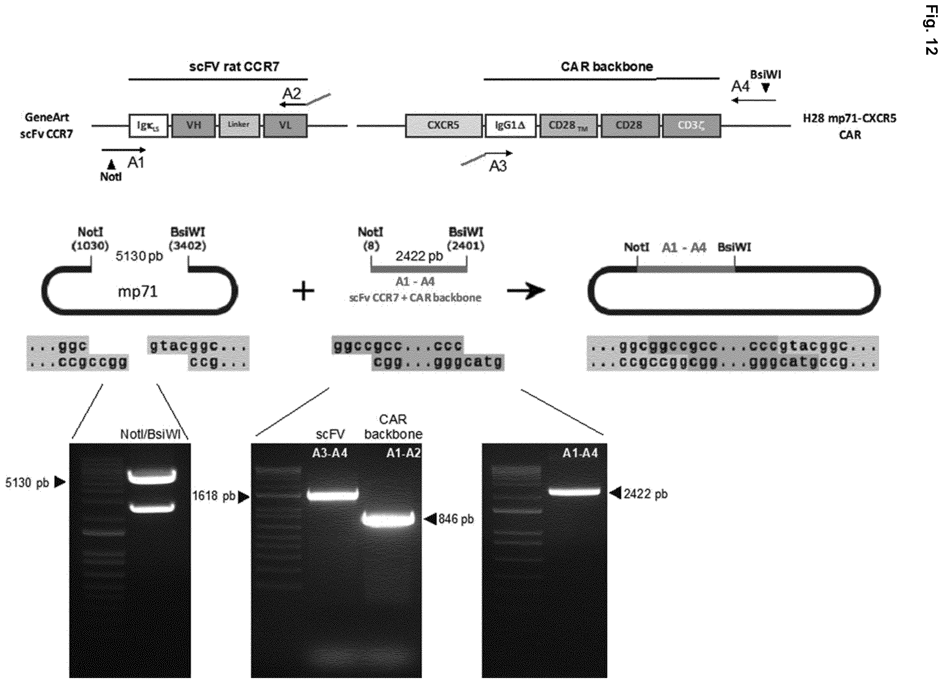

**Figure 12****:** Cloning and generation of the anti-CCR7 CAR construct. The illustration represents the cloning strategy of the synthesized 3D12 scFv from GeneArt^{®} gene synthesis into the MP71-CAR vector (top panel). Individual fragments were amplified via overlapping PCR, digested with Notl/BsiWI restriction enzymes, and ligated into the retroviral vector, harboring compatible ends (middle panel). Gel electrophoresis of digested MP71-CAR vector and amplified scFv and CAR backbone (fragments of interest indicated by arrows) are shown.

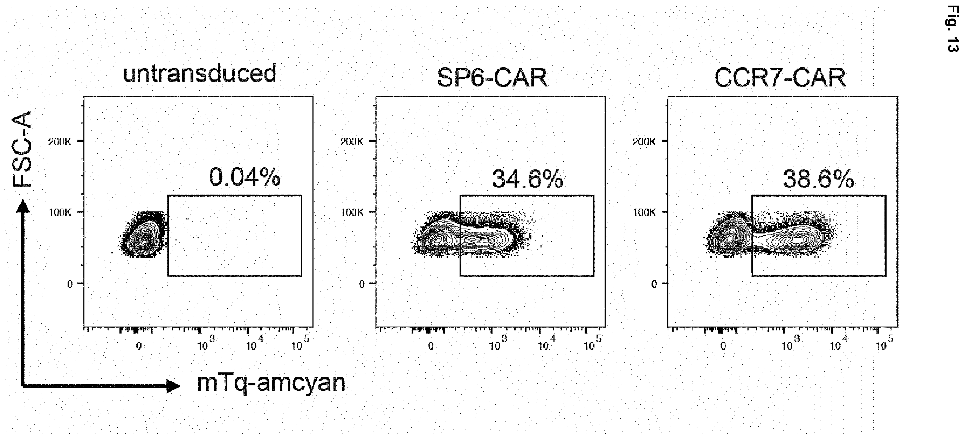

**Figure 13** CCR7 CAR expression on human primary NK cells following retroviral transduction. Flow cytometry of CAR expression, untransduced, construct SP6-CAR, CCR7-CAR. A fluorescent tag mTq was included in a bicistronic orientation, for easier detection.

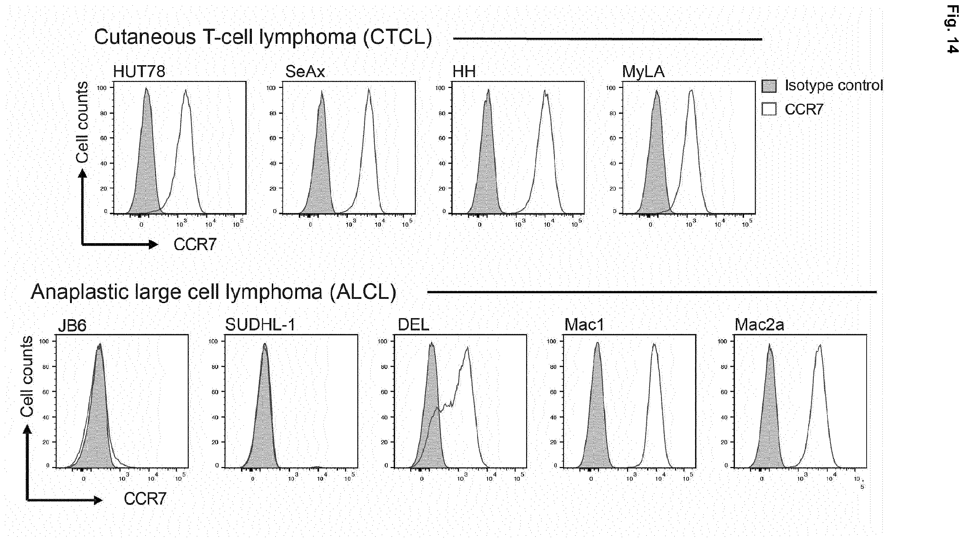

**Figure 14****:** CCR7 surface expression on T-NHL cell lines. Flow cytometry analysis of surface expression of the chemokine receptor CCR7 on T-NHL subentities. The cell lines were stained with the anti-CCR7mAB (red lines) or corresponding isotype control (filled grey lines). This antibody is the same binder used for the anti-CCR7 CAR design. Exemplary histograms show that CCR7 was expressed on the cutaneous T cell lymphoma (CTCLs) Hut-78, Se-Ax, HH, MyLa, and the anaplastic large cell lymphoma (ALCLs) DEL, Mac-1, and Mac-2a cell lines.

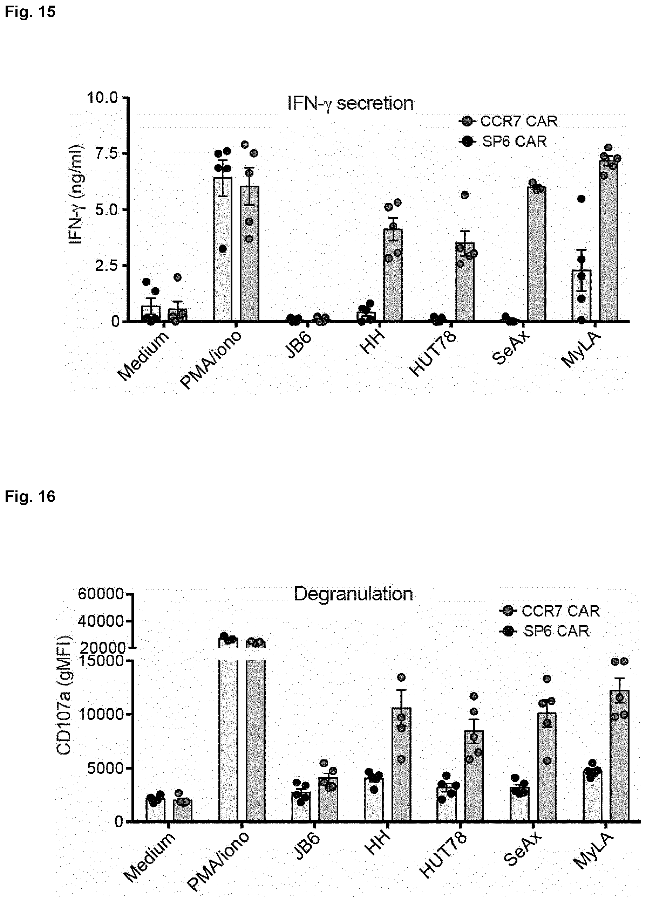

**Figure 15****:** Co-cultures of CAR-transduced human NK cells with different target cell lines show specific NK cell activation by distinct CCR7+ T-NHL and control cell lines. Functional *in vitro* co-cultivation and IFN-gamma ELISA were performed. The levels of IFN-gamma released are indicative of NK cell activation. CCR7-CAR expressing NK cells (red bars) and SP6-CAR NK cells (grey bars) were co-cultured with the target cell lines JB6, HH, HuT-78, Se-Ax, and MyLa cells. CCR7+ target cells induced specific IFN-gamma release in CCR7-CAR NK cells of the present invention.

**Figure 16****:** Functional *in vitro* co-cultivation and CD107a analysis as a measure for degranulation. Co-cultures of CAR-transduced human NK cells with different target cell lines show specific NK cell activation by distinct CCR7+ T-NHL and control cell lines. The levels of CD107a released are indicative of NK cell activation. CCR7-CAR expressing NK cells (red bars) and SP6-CAR NK cells (grey bars) were co-cultured with the target cell lines JB6, HH, HuT-78, Se-Ax, and MyLa cells. CCR7+ target cells induced specific CD107a release in CCR7-CAR NK cells of the present invention.

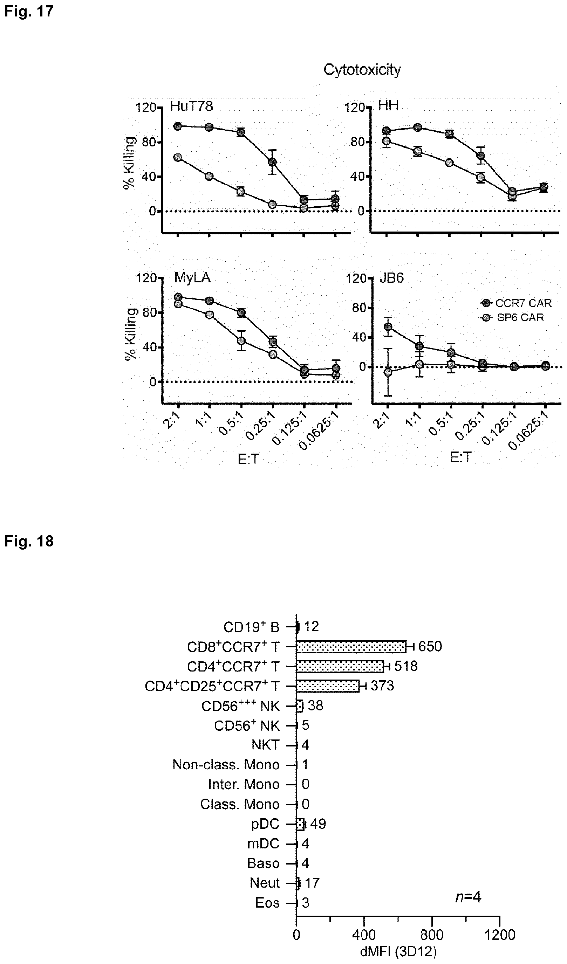

**Figure 17****:** Cytotoxicity assays reveal selective killing of CCR7+ cell lines. Essentially, no killing was seen in CCR7-negative cell lines. Two independent functional in vitro co-cultivations were performed. Target cell lines show lysis after treatment with the inventive CCR7-CAR, whereas cells not expressing CCR7 show no lysis.

**Figure 18****:** Quantitative determination of CCR7 density per cell on primary blood leukocyte cell subpopulations was performed by flow cytometry and a CCR7-specific antibody.

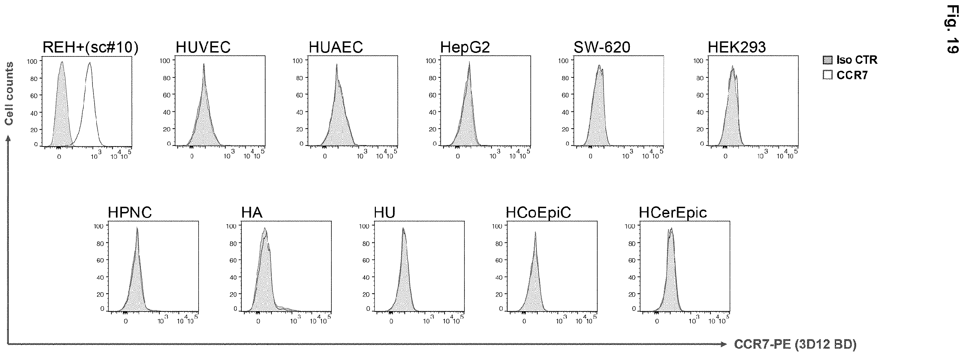

**Figure 19****:** CCR7 surface expression by anti-CCR7 immunostaining and flow cytometry analysis. To rule out that the CCR7-CAR NK cells of the present invention show cross-reactivity with healthy human tissues, CCR7 expression was assessed on a panel of primary cells derived from healthy human tissues. None of the tested primary human cells (HUVEC, human umbilical vein endothelial cells; HUAEC, human umbilical artery endothelial cells; HA, human astrocytes; HN, human neurons; HPNC, human perineurial cells; HCoEpiC, human colonic epithelial cells) showed CCR7 surface expression by anti-CCR7 immunostaining and flow cytometry analysis.

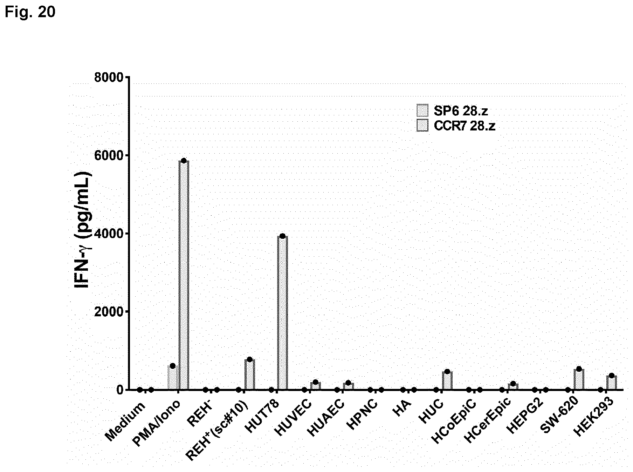

**Figure 20****:** Co-cultures of CAR-transduced human NK cells with CCR7-negative primary cells of different human tissues and the CCR7-cell line REH as targets show no off-target NK cell activation, whereas the CCR7-expressing T-NHL cell line HuT-78 and CCR7-transduced REH cell line mediate specific NK cell activation and serve as positive controls. Functional in vitro co-cultivation and IFN-gamma ELISA were performed. SP6 CAR NK cells (SP6, grey bars) and CCR7-CAR (red bars) expressing NK cells were co-cultured with the primary cells HUVECs, HUAECs, HAs, HNs, HPNCs, HCoEpiCs with the T-NHL cell line HuT-78. Lack of IFN-gamma release indicates an absence of specific NK cell activation in the presence of CCR7-negative primary cells and the CCR7-negative T-NHL cell line REH. CCR7-CAR NK cells of the present invention show specific IFN-gamma release in response to co-cultivation with CCR7-expressing HuT-78 target cells.

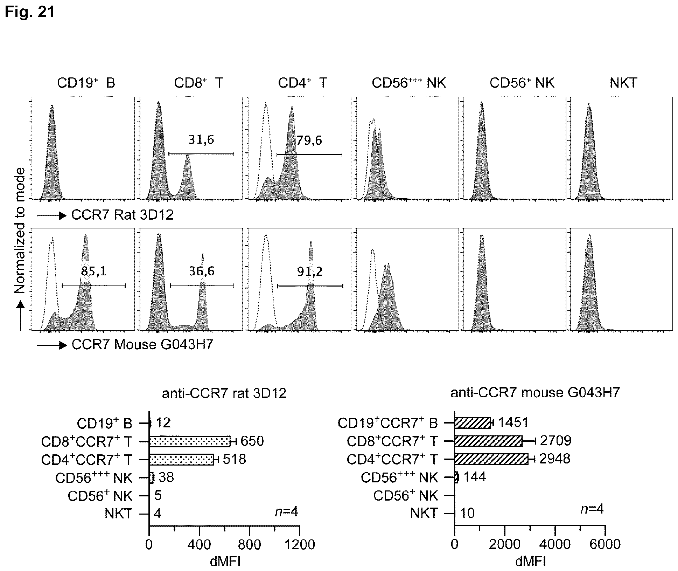

**Figure 21****:** Direct staining with the rat antibody 3D12 shows no positive signal for CCR7 expression on B cells but detects CCR7 positivity on T cells, whereas the mouse antibody G043H7 detects CCR7 on both types of lymphocytes. However, CCR7 expression can also be detected with the rat antibody 3D12 when the signal of the rat antibody is amplified (data not shown). Primary lymphocytes were stained with one of two anti-human CCR7 antibodies, rat 3D12 or mouse G043H7, and analyzed by flow cytometry. Peripheral blood was collected from healthy donors, erythrocytes were removed by ACK lysis, and Fc receptors were blocked with human serum prior to antibody staining. Cells were stained additionally with antibodies to identify the major lymphocyte cell types: B cells (CD19+ B), CD8 and CD4 T cells (CD8+ T, CD4+ T), CD56 high and low natural killer cells (CD56+++ NK, CD56+ NK), and natural killer T cells (NKT). In the case of a bimodal distribution, the CCR7-positive population was gated. Representative histograms from one donor are shown at the top, and plots summarizing the delta median fluorescence intensity (dMFI) of four donors are shown at the bottom.

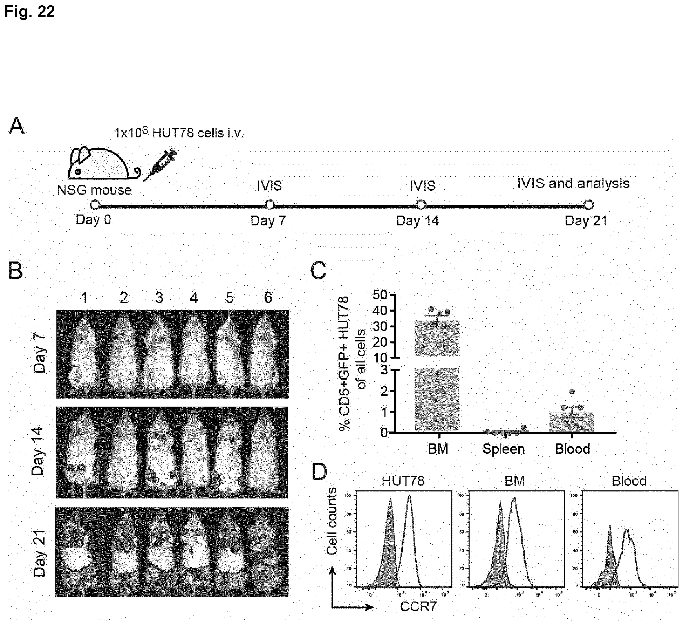

**Figure 22****:** Development of a T-NHL xenograft model. (A, B) HuT-78 cutaneous T lymphoma cells (1×10⁶ cells) stably expressing the GFP-Luc construct were adoptively transferred intravenously into NSG mice (n=6) and monitored over a time course of 21 days by IVIS imaging. At day 21 after the transfer, the (C) percentage of HuT-78 cells of all bone marrow or peripheral leukocytes was determined via anti-human CD5 staining and GFP-positivity analyzed by flow cytometry. Exemplary histograms in (D) demonstrate CCR7 expression level of pre-gated CD5+GFP+ tumor cells stained against CCR7.

### EXAMPLES

The invention is demonstrated by way of the examples disclosed below. The examples provide technical support for and a more detailed description of potentially preferred, non-limiting embodiments of the invention.

In order to demonstrate the functionality and beneficial properties of the CAR described herein, the following examples are to be considered:
- Functional in vitro testing of anti-CCR7 CAR-NK cells
- Cytotoxicity assays
- Development of a T-NHL xenograft model.

### Example 1: Generation of the anti-CCR7 CAR construct

In brief, as shown in Fig. 12, the antibody 3D12 scFv from GeneArt^{®} gene synthesis is synthesized into the MP71-CAR vector (top panel). Individual fragments were amplified through overlapping PCR and digested with Notl/BsiWI restriction enzymes. The resulting fragment was ligated into the retroviral vector, harboring compatible ends (middle panel). Gel electrophoresis of digested MP71-CAR vector, amplified scFV (A1-A2), CAR backbone (A3-A4), and fused insert (A1-A4). Fragments of interest are highlighted by arrows.

### Method/Protocol

Variable heavy- and light-chain sequences of the rat anti-human CCR7 antibody (clone 3D12) were used to design scFv sequences. To construct the scFv, the VH sequence was fused to an immunoglobulin K (IgK) leader sequence and connected to the VL sequence through a Whitlow linker (18 aa). The CAR backbone consists of an IgG1 (266-503 aa) spacer with mutated Fc-γ binding regions, followed by a CD28 transmembrane region, the cytoplasmic portion of the CD28 molecule, and CD3ζ activation domain. The full sequence encoding the scFv was codon optimized and synthesized by GeneArt (Life Technologies, Darmstadt, Germany), and amplified with a pair of primers harboring the enzymatic restriction sites Notl. The sequence of the CAR backbone was amplified from the MP71-CXCR5 CAR construct (Bunse et al., 2021) with a pair of primers harboring the enzymatic restriction sites BsiW. The two fragments were fused via overlapping PCR. The MP71-CXCR5 CAR was enzymatically digested at the Notl and BsiW restriction sites to obtain the MP71 retroviral vector scaffold. The CCR7 scFV-CAR insert harboring Notl/BsiW compatible sticky ends was subsequently cloned into the MP71 retroviral vector scaffold.

### Example 2: Functional in vitro testing of anti-CCR7 CAR-NK cells

In brief, as shown in Fig. 14, the cell lines were stained with the anti-CCR7mAB (red lines) or corresponding isotype control (filled grey lines). This antibody is the same binder used for the anti-CCR7 CAR design. Exemplary histograms show that CCR7 was expressed on the cutaneous T cell lymphoma (CTCLs) HuT-78, Se-Ax, HH, MyLa, and the anaplastic large cell lymphoma (ALCLs) DEL, Mac1, and Mac2a cell lines.

Moreover, as seen in Fig. 15, co-cultures of CAR-transduced human NK cells with different target cell lines show specific NK cell activation by distinct CCR7+ T-NHL and control cell lines. Functional *in vitro* co-cultivation and IFN-gamma ELISA were performed. The levels of IFN-gamma released are indicative of NK cell activation. CCR7-CAR expressing NK cells (red bars) and SP6-CAR NK cells (grey bars) were co-cultured with the target cell lines JB6, HH, HuT-78, Se-Ax, and MyLa cells. CCR7+ target cells induced specific IFN-gamma release in CCR7-CAR NK cells of the present invention.

Fig. 16 depicts the functional *in vitro* co-cultivation and CD107a analysis as a measure for degranulation. Co-cultures of CAR-transduced human NK cells with different target cell lines show specific NK cell activation by distinct CCR7+ T-NHL and control cell lines. The levels of CD107a released are indicative of NK cell activation. CCR7-CAR expressing NK cells (red bars) and SP6-CAR NK cells (grey bars) were co-cultured with the target cell lines JB6, HH, HuT-78, Se-Ax, and MyLa cells. CCR7+ target cells induced specific CD107a release in CCR7-CAR NK cells of the present invention.

### Method/Protocol

### Degranulation assay

CAR-engineered NK-92 cells were co-cultured with T-NHL cell lines in a 96-well U-bottom plate at 1:1 ratio (5×10⁴ cells per well each) for 2 hours at 37°C. Anti-human CD107a-PE antibody (H4A3, Biolegend) was added directly to the co-culture. At the end of the stimulation period, cells were washed in ice-cold FACS buffer, blocked with 5% human serum in FACS buffer for 10 minutes at RT, and stained with anti-human CD56-AF647 (5.1H11, BioLegend). Degranulation rate was measured as gMFI of CD107a-positive CD56⁺mTq⁺ NK-92 or primary NK cells. Maximum degranulation was induced by stimulation of effector cells with phorbol myristate acetate (PMA) (5 ng/ml)/ ionomycin (lono; 1 µM), and minimum degranulation represents effectors incubated without target cells.

### IFN-gamma secretion

5×10⁴ CAR NK-92 cells were co-incubated with an equal number of T-NHL cells for 16 hours at 37°C in a U-bottom 96-well plate. After cell removal, IFN-gamma concentration in the supernatant was assessed using the BD OptEIA^{™} ELISA kit on Nunc MaxiSorp 96-well plates. The standard series was performed in duplicates, starting at 8 ng. Data were acquired using a PowerWave X Select microplate UV-VIS reader (BioTek Instruments, USA). Maximal release was induced by phorbol myristate acetate (PMA; 5 ng/ml / ionomycin (1 µM) stimulation of T cells, while T cells incubated without target cells represent minimum release.

**Table 2. CCR7 expression on distinct human cell lines**

| **Cell line** | **Origin** | **CCR7-positivity** |
|---|---|---|
| DEL | Anaplastic Large Cell Lymphoma (ALCL), t (2;5)-positive | yes |
| DL-40 | Anaplastic Large Cell Lymphoma (ALCL), t (2;5)-positive | yes |
| FE-DP | Anaplastic Large Cell Lymphoma (ALCL), t (2;5)-negative | yes |
| Mac-1 | Anaplastic Large Cell Lymphoma (ALCL), t (2;5)-negative | yes |
| JB6 | Anaplastic Large Cell Lymphoma (ALCL), t (2;5)-positive | no |
| SU-DHL-1 | Anaplastic Large Cell Lymphoma (ALCL), t (2;5)-positive | no |
| Karpas-299 | Anaplastic Large Cell Lymphoma (ALCL), t (2;5)-positive | no |
| Mac-2a | Anaplastic Large Cell Lymphoma (ALCL), t (2;5)-negative | yes |
| HuT-78 | Cutaneous T cell Lymphoma, Sezary Sindrome (SS)-derived | yes |
| Se-Ax | Cutaneous T cell Lymphoma, Sezary Sindrome (SS)-derived | yes |
| MyLa | Cutaneous T cell Lymphoma, Mycosis Fungoides (MF)-derived | yes |
| HH | Cutaneous T cell Lymphoma, Mycosis Fungoides (MF)-derived | yes |
| L-1236 | malignant mononuclear Hodgkin-/multinuclear Reed-Sternberg (HRS) cells | yes |
| L-591 | malignant mononuclear Hodgkin-/multinuclear Reed-Sternberg (HRS) cells | yes |
| REH | B acute lymphoblastic leukemia (B-ALL) | no |
| JeKo-1 | Mantle Cell Lymphoma (MCL), B-NHL | yes |
| DOHH-2 | immunoblastic B cell lymphoma progressed from follicular centroblastic/centrocytic lymphoma (FL) | no |
| OCI-Ly7 | diffuse large B cell lymphoma (DLBCL), germinal center type | no |
| SU-DHL4 | diffuse large B cell lymphoma (DLBCL), germinal center type | no |
| SC-1 | B follicular lymphoma (B-NHL) | yes |
| Jurkat | T cell acute lymphoblastic leukemia (T-ALL) | no |
| Raji | Burkitt's lymphoma | yes |
| NK-92 | Natural killer cell lymphoblastic leukemia/lymphoma | no |
| YTS | Natural killer cell lymphoblastic leukemia/lymphoma | no |
| JVM-3 | Chronic B cell leukemia (B-CLL) | yes |
| MEC-1 | Chronic B cell leukemia (B-CLL) | no |
| HepG2 | Hepatocellular carcinoma cell | no |
| SW-620 | Colon adenocarcinoma cell | no |
| HEK293 | Embryonic kidney cell | no |
| HEK-CCR7 | Embryonic kidney cell transfected with CCR7 | yes |

### Example 3: Cytotoxicity assay and specificity of CCR7 CAR.

As shown in Fig. 17, cytotoxicity assays reveal selective killing of CCR7+ cell lines. Essentially, no killing was seen in CCR7-negative cell lines. Two to five independent functional in vitro co-cultivations were performed. Target cell lines show lysis after treatment with the inventive CCR7-CAR, whereas cells not expressing CCR7 show no lysis.

Quantitative determination of CCR7 density per cell on primary blood leukocyte cell subpopulations was performed by flow cytometry and a CCR7-specific antibody, as seen in Fig. 18.

To rule out that the CCR7-CAR NK cells of the present invention show cross-reactivity with healthy human tissues, CCR7 expression was assessed on a panel of primary cells derived from healthy human tissues (Fig. 19 and Table 3). None of the tested primary human cells (HUVEC, human umbilical vein endothelial cells; HUAEC, human umbilical artery endothelial cells; HA, human astrocytes; HN, human neurons; HPNC, human perineurial cells; HCoEpiC, human colonic epithelial cells) showed CCR7 surface expression by anti-CCR7 immunostaining and flow cytometry analysis.

**Table 3. CCR7 surface expression of primary human cells**

| **Primary cells** | **Origin** | **CCR7-positivity** |
|---|---|---|
| HUVEC | Human Umbilical Vein Endothelial Cells | no |
| HUAEC | Human Umbilical Artery Endothelial Cells | no |
| HA | Human Astrocytes | no |
| HPNC | Human Perineurial Cells | no |
| HCoEpiC | Human Colonic Epithelial Cells | no |
| HCerEpic | Human Cervical Epithelium Cells | no |
| HU | Human urothelial cells | no |

As shown in Figure 20, co-cultures of CAR-transduced human NK cells with CCR7-negative primary cells of different human tissues and the CCR7-cell line REH as targets show no off-target NK cell activation, whereas the CCR7-expressing T-NHL cell line HuT-78 and CCR7-transduced REH cell line mediate specific NK cell activation and serve as positive controls. Functional *in vitro* co-cultivation and IFN-gamma ELISA were performed. SP6 CAR NK cells (SP6, grey bars) and CCR7-CAR (red bars) expressing NK cells were co-cultured with the primary cells HUVECs, HUAECs, HAs, HNs, HPNCs, HCoEpiCs with the T-NHL cell line HUT78. Lack of IFN-gamma release indicates an absence of specific NK cell activation in the presence of CCR7-negative primary cells and the CCR7-negative T-NHL cell line REH. CCR7-CAR NK cells of the present invention show specific IFN-gamma release in response to co-cultivation with CCR7-expressing HUT78 target cells.

As shown in Figure 21, direct staining with the rat antibody 3D12 shows no positive signal for CCR7 expression on B cells but detects CCR7 positivity on T cells, whereas the mouse antibody G043H7 detects CCR7 on both types of lymphocytes. However, CCR7 expression can also be detected with the rat antibody 3D12 when the signal of the rat antibody is amplified (data not shown). Primary lymphocytes were stained with one of two anti-human CCR7 antibodies, rat 3D12 or mouse G043H7, and analyzed by flow cytometry. Peripheral blood was collected from healthy donors, erythrocytes were removed by ACK lysis, and Fc receptors were blocked with human serum prior to antibody staining. Cells were stained additionally with antibodies to identify the major lymphocyte cell types: B cells (CD19+ B), CD8 and CD4 T cells (CD8+ T, CD4+ T), CD56 high and low natural killer cells (CD56+++ NK, CD56+ NK), and natural killer T cells (NKT). In the case of a bimodal distribution, the CCR7-positive population was gated. Representative histograms from one donor are shown at the top, and plots summarizing the delta median fluorescence intensity (dMFI) of four donors are shown at the bottom.

### Method/Protocol

The cytolytic activity of CAR NK-92 cells was analyzed by flow cytometry. CAR-engineered NK-92 were seeded in 96-well U-bottom plates and mixed with a fixed number of 5×10⁴ GFP+ target cells. Effector-to-target ratios were titrated in the range of 5:1, 1:1, 0.5:1, 0.25:1, and 0.125:1. Co-cultures in RPMI medium were kept for 16 hours and stopped by adding ice-cold FACS buffer. Cells were washed and resuspended in 5% of human serum in FACS buffer for 10 min. To distinguish effector and target cells, anti-human CD56-AF647 (5.1H11, BioLegend) antibody in combination with 7-AAD for dead cell exclusion were used. Samples were analyzed on a FACSCanto II Cell Analyzer (BD Biosciences) and further analyzed with FlowJo v. 10.0.8 software. The relative percentage of specific target cell killing is determined using the formula: %= (1 - (tumor cells in coculture / R)) × 100. 'R' depends on the E:T ratio: 33,3 (2:1); 50 (1:1); 66,7 (0,5:1); 80 (0,25:1); 89,9: (0,125:1); 94,1(0,0625:1).

### Example 4: Development of a T-NHL xenograft model

Figure 22 demonstrates the development of a T-NHL xenograft model. As seen in Figure 22A ad B, HUT-78 cutaneous T lymphoma cells (1×10⁶ cells) stably expressing the GFP-Luc construct were adoptively transferred intravenously into NSG mice (n=6) and monitored over a time course of 21 days by IVIS imaging. At day 21 after the transfer, the (C) percentage of HuT-78 cells of all bone marrow or peripheral leukocytes was determined via anti-human CD5 staining and GFP-positivity analyzed by flow cytometry. Exemplary histograms in (D) demonstrate CCR7 expression level of pre-gated CD5+GFP+ tumor cells stained against CCR7.

### Method/Protocol

In brief, eight to ten week old NSG mice (NOD.Cg-Prkdc^{scid} II2rg^{tm1 Wjl}/SzJ, Jackson laboratories) were intravenously injected in the tail vein with HuT-78 cells expressing firefly luciferase and eGFP (1×10⁶/mouse/100 µl). Tumor growth was monitored weekly with an IVIS Spectrum imaging system (Perkin Elmer). Bone marrow, spleen, and blood were collected and tumor load was determined as percentages of CD5+ GFP+ cells in each compartment by FACS analysis.

### Xenograft mouse model

For xenograft experiments, eight to ten week old NSG mice (NOD.Cg-Prkdc^{scid} II2rg^{tm1 Wjl}/SzJ, Jackson laboratories) were intravenously injected in the tail vein with HuT-78 cells expressing firefly luciferase and eGFP (1×10⁶/mouse/100 µl). These mice are specifically suited for a xenotransplantation because they support human cell growth by cytokine provision and have no T, B, and NK cells for rejection of the transplant. The T-NHL cell line HUT78 is also stably transduced with a firefly-luciferase gene that allows for in vivo tumor cell detection by applying luciferin. Progression and distribution of tumors injected are monitored by bioluminescence imaging using the IVIS-system (Perkin Elmer).

### In vivo imaging

Mice were anesthetized with 2% isoflurane in the XGI-8 Gas Anesthesia System (Caliper Life Science) and injected with luciferin (150 µg/mouse in PBS). After 10 minutes, they were transferred to the heated imaging platform (Xenogen IVIS 2000) for imaging at varying exposure times (1-300 s). Images were captured with small binning, and Subsequent analysis was conducted using Livinglmage 4.5 software from Caliper Life Science.

### Isolation of bone marrow, spleen and blood

At the experiment's endpoint, mice were euthanized with an isoflurane overdose. BM, spleen, and blood were collected for flow cytometric analysis. For BM analysis, femurs were dissected and flushed with PBS. Spleens were homogenized in FACS buffer. Isolated BM and spleen cells were filtered, centrifuged, and subjected to red blood cell lysis. PBMC from blood were obtained through heart puncture, mixed with 50 µl 0.5 M EDTA, and processed for FACS analysis. Tumor load was determined as percentages of CD45+ GFP+ cells in each compartment by flow cytometry analysis.

### REFERENCES

1. Legler DF, Uetz-von Allmen E, Hauser MA. CCR7: roles in cancer cell dissemination, migration and metastasis formation. Int J Biochem Cell Biol. 2014 Sep;54:78-82. doi: 10.1016/j.biocel.2014.07.002. Epub 2014 Jul 11. PMID: 25019368.
2. Cuesta-Mateos C, Terrón F, Herling M. CCR7 in Blood Cancers - Review of Its Pathophysiological Roles and the Potential as a Therapeutic Target. Front Oncol. 2021 Oct 29;11:736758. doi: 10.3389/fonc.2021.736758. PMID: 34778050; PMCID: PMC8589249.
3. Jogalekar MP, Rajendran RL, Khan F, Dmello C, Gangadaran P, Ahn BC. CAR T-Cell-Based gene therapy for cancers: new perspectives, challenges, and clinical developments. Front Immunol. 2022 Jul 22;13:925985. doi: 10.3389/fimmu.2022.925985. PMID: 35936003; PMCID: PMC9355792.
4. M 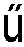 zes G, Sipos F. CAR-Based Therapy for Autoimmune Diseases: A Novel Powerful Option. Cells. 2023 Jun 2;12(11):1534. doi: 10.3390/cells12111534. PMID: 37296654; PMCID: PMC10252902.
5. Fu H, Liang Y, Zhong X, Pan Z, Huang L, Zhang H, Xu Y, Zhou W, Liu Z. Codon optimization with deep learning to enhance protein expression. Sci Rep. 2020 Oct 19;10(1):17617. doi: 10.1038/s41598-020-74091-z. PMID: 33077783; PMCID: PMC7572362.
6. Li Y, Hermanson DL, Moriarity BS, Kaufman DS. Human iPSC-Derived Natural Killer Cells Engineered with Chimeric Antigen Receptors Enhance Anti-tumor Activity. Cell Stem Cell. 2018 Aug 2;23(2):181-192.e5. doi: 10.1016/j.stem.2018.06.002. Epub 2018 Jun 28. PMID: 30082067; PMCID: PMC6084450.
7. Laskowski TJ, Biederstädt A, Rezvani K. Natural killer cells in antitumour adoptive cell immunotherapy. Nat Rev Cancer. 2022 Oct;22(10):557-575. doi: 10.1038/s41568-022-00491-0. Epub 2022 Jul 25. PMID: 35879429; PMCID: PMC9309992.
8. Martin J. Raftery, Alexander Sebastian Franzén, Gabriele Pecher. CAR NK Cells: The Future Is Now. Annual Review of Cancer Biology 2023 7:1, 229-246
9. Kärre K, Ljunggren HG, Piontek G, Kiessling R. Selective rejection of H-2-deficient lymphoma variants suggests alternative immune defence strategy. Nature. 1986 Feb 20-26;319(6055):675-8. doi: 10.1038/319675a0. PMID: 3951539.

## Claims

1. A chimeric antigen receptor polypeptide (CAR), comprising:
i. an extracellular antigen-binding domain, comprising an antibody or antibody fragment that binds C-C chemokine receptor type 7 (CCR7) protein,
ii. a transmembrane domain, and
iii. an intracellular domain.

2. The chimeric antigen receptor (CAR) polypeptide according to the preceding claim, wherein the antigen-binding domain comprises a variable heavy chain (VH), said VH comprising:
- a heavy chain complementary determining region 1 (H-CDR1) according to SEQ ID NO 1 (GFTFSNAA),
- a heavy chain complementary determining region 2 (H-CDR2) according to SEQ ID NO 2 (IRTKPNNFAT), and
- a heavy chain complementary determining region 3 (H-CDR3) according to SEQ ID NO 3 (TAGKDYFAY),
and a variable light chain (VL), said VL comprising:
- a light chain complementary determining region 1 (L-CDR1) according to SEQ ID NO 4 (KSLLFSDGKTY),
- a light chain complementary determining region 2 (L-CDR2) according to SEQ ID NO 5 (WMS), and
- a light chain complementary determining region 3 (L-CDR3) according to SEQ ID NO 6 (QQFLEFPFT).

3. The chimeric antigen receptor (CAR) polypeptide according to any one of the preceding claims, comprising a VH domain with at least 70% sequence identity, preferably at least 80%, 85%, 90%, or 95% sequence identity, to SEQ ID NO 7: 
X1VQLVESGGGLVQPX2X3SLKX4SCAASGFTFSX5X6AMX7WVRQAX8GKGLEWVX9RIR
X10KX11NX12X13ATX14YAX15SVKGRFTISRDDSKX16X17X18YLQMX19X20LKTEDTAX
21YYCTAGKDYFAYWGQGTLVTVSS,
wherein X1: A or E; X2: G or K; X3: G or E; X4: L or I, X5: N or G; X6: A or S; X7: H or Y; X8: Sor P; X9: G or A; X10: T orS; X11: P or A; X12: NorS;X13: F or Y; X14: Y or A; X15: D or A; X16: Nor S; X17: M or T; X18: A or V; X19: N or D; X20: K or S; X21: V or M;
and a VL domain with at least 70% sequence identity, preferably at least 80%, 85%, 90%, or 95% sequence identity, to SEQ ID NO 8: 
DIVMTQX1X2LX3X4PVX5X6GEX7ASIX8CX9SSX10SLLX11SDGX12TYLX13WYLQX14PG
QSPQX15LIYX16X17SX18RASGVX19DRFSGSGSGTDFTLKISRVEAEDVGVYYCX20QX2
1X22EFPFTFGSGTKLEIKR
wherein X1:T or G; X2: P or A; X3: S or P; X4: L or N; X5: T or P; X6: PorS;X7: PorS;X8: S or T; X9: R or Q; X10: K or Q; X11: F or D; X12: K or N; X13: D or N; X14: KorR;X15: L or F; X16: W or T; X17: M or L; X18: Y or T; X19: P or S; X20: Q or M; X21: For R; X22: L or I .

4. The chimeric antigen receptor (CAR) polypeptide according to any one of the preceding claims, comprising a VH domain according to one of SEQ ID NO 9 to SEQ ID NO 16, and a VL domain according to one of SEQ ID NO 17 to SEQ ID NO 27.

5. The chimeric antigen receptor (CAR) polypeptide according to any one of the preceding claims, wherein when said CAR is expressed in a genetically modified immune cell, preferably a natural killer (NK) or T cell, said immune cell binds CCR7 on the surface of a CCR7-expressing cell and is activated, thereby inducing cytotoxic activity against said CCR7-expressing cell, wherein the CCR7-expressing cell is preferably a DEL, DL-40, FE-DP, Mac-1, Mac-2a, HuT-78, Se-Ax, MyLa, HH, L-1236, L-591, SC-1, Raji, or JVM-3 cell line.

6. The chimeric antigen receptor (CAR) polypeptide according to any one of the preceding claims:
- wherein the extracellular antigen-binding domain comprises a linker polypeptide positioned between the VH and VL domains, wherein said linker is preferably a Whitlow linker (SEQ ID NO 28; GSTSGSGKPGSGEGSTKG) or linkers with at least 80% sequence identity to SEQ ID NO 28; and/or
- comprising additionally a spacer polypeptide positioned between the extracellular antigen-binding domain and the transmembrane domain, wherein said spacer is preferably an IgG1 spacer (SEQ ID NO 29; PAEPKSPDKTHTCPPCPAPPVAGPSVFLFPPKPKDTLMIARTPEVTCVVVDVSHED PEVKFNWYVOGVEVHNAKTKPREEQYNSTYRVVSVLTVLHQOWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNH YTQKSLSLSPGKKDPK), or a spacer with at least 80% sequence identity to SEQ ID NO 29; and/or
- wherein the transmembrane domain is a CD28 domain (SEQ ID NO 30; FWVLVVVGGVLACYS LLVTVAF II FWV), or transmembrane domain with at least 80% sequence identity to SEQ ID NO 30; and/or
- wherein the intracellular domain comprises a CD28 co-stimulatory domain (SEQ ID NO 31; RSKRSRLLHSDYMNMTPRRPGPTRKHYQPYAPPRDFAAYRSL), or a co-stimulatory domain with at least 80% sequence identity to SEQ ID NO 31; and/or
- comprising additionally a signaling domain (activation domain), wherein said signaling domain is preferably a CD3zeta signaling domain (SEQ ID NO 32; LRVKFSRSADAPAYQQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR) or a signaling domain with at least 80% sequence identity to SEQ ID NO 32; and/or
- comprising additionally a signal peptide, wherein said signal peptide is preferably an IgK signal peptide (SEQ ID NO. 33; MDFQVQIFSFLLISASVIMSR) or a signal peptide with at least 80% sequence identity to SEQ ID NO 33.

7. The chimeric antigen receptor (CAR) polypeptide according to any one of the preceding claims, comprising or consisting of a sequence according to any one of SEQ ID NO 34.

8. An isolated nucleic acid molecule, preferably in the form of a vector, such as a retroviral vector, lentiviral vector or transposon, encoding a chimeric antigen receptor (CAR) polypeptide according to any one of the preceding claims.

9. A genetically modified immune cell comprising a nucleic acid molecule according to claim 8 and/or expressing a CAR according to any one of claims 1 to 7.

10. The genetically modified immune cell according to claim 9, wherein the cell is a natural killer (NK) cell.

11. The genetically modified immune cell according to any one of claims 9 or 10 **for use in the treatment of a medical disorder** associated with the presence of pathogenic cells expressing CCR7, preferably semi-mature or mature dendritic cells, monocytes/macrophages, naive B cells and T cells, including T regulatory and central memory T cells.

12. The genetically modified immune cell for use according to claim 11, wherein the medical disorder is T cell lymphoma, preferably T cell non-Hodgkin's lymphoma (T-NHL).

13. The genetically modified immune cell for use according to claim 11, wherein the medical disorder is a T cell non-Hodgkin's lymphoma, with or without a leukemic tumor cell dissemination.

14. The genetically modified immune cell for use according to claim 11, wherein the medical disorder is:
- a B cell-derived lymphoproliferative disorder, selected from the group consisting of chronic lymphatic leukemia (CLL), mantle cell lymphoma (MCL) and classical Hodgkin lymphoma (cHD); or
- a T cell-derived lymphoproliferative disorder, selected from the group consisting of T-cell prolymphocytic leukemia (T-PLL) and T cell leukemia central nervous system (CNS) infiltration.

15. The genetically modified immune cell for use according to claim 11, wherein the medical disorder is an autoimmune disease.
